# EUROPEAN PATENT APPLICATION

(11) **EP 2 944 190 A1**
(43) Date of publication of application: **18.11.2015**
(21) Application number: 14168494.4
(22) Date of filing: 15.05.2014
(51) Int. Cl.: A01H 5/10, C08B 30/00, C07K 14/415, C12N 15/82

(54) **Means and methods for modifying the amylose content in plant starch**

(71) Applicant: ETH Zurich, 8092 Zurich (CH)
(72) Inventor: Seung, David, 8057 Zürich (CH); Zeemann, Samuel, 8032 Zürich (CH); Soyk, Sebastian, 8045 Zürich (CH)

(57) **Abstract**

Described are means and methods for modifying the amylose content in plant starch, in particular by making use of the PTST (Protein Transporting to Starch) protein. Also described are plants that are modified so as to have a decreased or increased amylose content.

## Description

The present invention relates to means and methods for modifying the amylose content in plant starch, in particular by making use of the PTST (Protein Transport to Starch) protein. The present invention also relates to plants modified so as to have a decreased or increased amylose content.

Plants produce starch because it is an effective way to store the carbon that they fix during photosynthesis. For society, starch is a cheap, renewable plant product and has many industrial uses. It is the major component of our staple foods and a feedstock for bio-ethanol production.

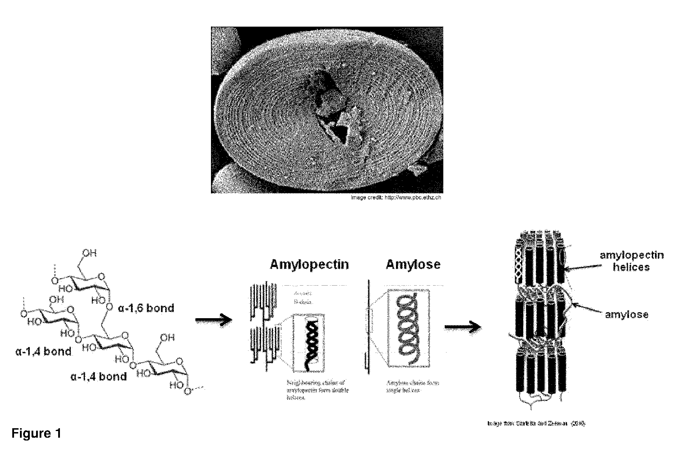

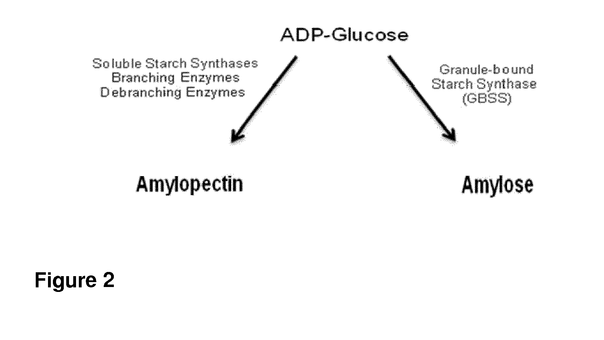

Starch is synthesized during the day within the chloroplasts in the leaf mesophyll cells, i.e. the site where photosynthesis takes place. The plant degrades this starch during the night when photosynthesis is not possible. The degradation of starch during the night is critical for fuelling growth and cellular processes in the dark. Starch is also found in seeds, grains and storage organs (such as tubers). The starch in these organs act as an energy reserve that powers the process of germination.
In its native form, starch exists as insoluble, semi-crystalline granules (see Figure 1). However, at the molecular level, it is composed almost completely of polymerised glucose units. There are α-1,4 glucosidic bonds between glucose units in linear chains, and α-1,6 glucosidic bonds between glucose units at branch points that link these linear chains (see Figure 1).
Two distinct polymers exist within the starch granule - amylose and amylopectin. Amylose is composed of long linear chains with few branch points. Amylopectin has shorter chains and many more branch points. Adjacent chains in amylopectin form double helices, and these double helices can pack together, resulting in the crystalline regions of starch. Amylose primarily exists within the less-crystalline (amorphous) zones inside the starch granule. This includes the space between the clusters of amylopectin helices (see Figure 1).
The synthesis of amylopectin requires the coordinated activities of at least three classes of enzymes. The soluble starch synthases elongate the chains of amylopectin, using the glucose donor molecule, ADP-glucose. To achieve the organised branching pattern, both branching enzymes and debranching enzymes are required. There is only one enzyme that is known to be exclusively involved in amylose biosynthesis. This is the granule-bound starch synthase (GBSS), which uses ADP-glucose to elongate amylose (see Figure 2). Unlike amylopectin, amylose is not essential for the formation of crystalline starch granules, and mutant plants lacking GBSS activity produce a starch containing only amylopectin.
The GBSS protein is exclusively 'granule bound', and previous studies have been unable to detect 'free' GBSS in the plastid stroma. This is in contrast to the soluble starch synthases, which are primarily found as a soluble form in the stroma. The localisation of GBSS within the starch granule is consistent with the current models of amylose synthesis, which suggest that amylose is synthesised inside pre-existing granules, i.e. within a matrix of semi-crystalline amylopectin. Amylopectin synthesis, on the other hand, is thought to occur at the granule surface and amylopectin molecules are radially orientated within the granule.

The relative amount of amylose and amylopectin is a major determinant of the physicochemical properties of starch. These properties are increasingly exploited in the agri-food sector (Delcour et al., 2010, Ann. Rev. Food Sci. Technol. 1, 87-111) and in many non-food manufacturing processes (e.g. the paper and packaging industries, biodegradable plastic production etc.). The amylose content is one of the most important parameters that determine starch quality for many industries. Quality is defined as the fitness for a defined end use. Low amylose content is desirable for some applications, whereas high amylose is desirable for others. Amylose content varies naturally depending on botanical source. For example, starch in Arabidopsis leaves contains 8-10% amylose; starches in most cereals and storage organs contain about 20-30% amylose.
The most important physical changes that take place during industrial processing of native starches are the swelling of the granules upon heating in an excess of water and subsequent solubilization of amylose and amylopectin ("gelatinization"). During this process, amylose diffuses out of the swollen amylopectin-containing granule and, on cooling, forms a continuous gel phase. Swollen amylopectin-enriched granules aggregate into gel particles, generating a viscous solution. This two-phase structure, called starch paste, is desired in many applications where processed starches are used as thickeners or binders.

The amylose/amylopectin ratio affects starch gelatinization and recrystallization properties. During processing, amylopectin forms viscous solutions that are stable in water at room temperature for days. In contrast, amylose forms a gel that is stable in solution at temperatures greater than 60-70°C, but on cooling it will rapidly aggregate or crystallize ("retrogradation"). Thus, low amylose starches are desirable in processed foods as they confer freeze-thaw stability. In contrast, high amylose starches (ie: >40%) or starches that have a lower degree of amylopectin branching, are characterized by higher gelatinization temperatures and a lower peak viscosities. Due to its high gelling strength and the film-forming ability, these starches have been extensively used in the production of biodegradable plastics, and have also been used in the production of corrugated boards as a coating and adhesive.
Starches with no amylose, such as waxy corn starch, have been extensively used by the food industry for many years. Its clear, sticky gelling properties have made it desirable for use in certain food applications. Genetically modified potato plants ("Amflora"; BASF) in which GBSS is inactivated produce a starch which contains no amylose, and was initially marketed towards the paper industry where clear, consistent gels are required.
Starch composition is also of importance for human health. In particular, starches that are resistant to rapid degradation in the gut confer improved health as they have a lower glycemic index and promote a healthy gut flora - serving as a source of dietary fiber. High amylose tends to confer such resistant properties to starch. Accordingly, starches with high amylose content (ie: >40%) have also received market interest, since high amylose starches are less-readily digested in the human gut, and its consumption may contribute to our dietary fiber intake.

The optimal use of starch for various applications requires the development of crops with more starch, or with altered starch composition/structure to provide functional diversity. Changing plants at the genetic level to alter genes encoding starch metabolizing enzymes can result in starches synthesized in the plant with altered properties. Plants with altered amylose content have been identified in a number of species. Initially, naturally-occurring low amylose maize and rice varieties were selected by farmers and breeders as they delivered consumer-preferred properties upon cooking compared with the conventional crop. The only known gene to be affected in such varieties encodes granule-bound starch synthase (GBSS - also called the waxy gene as maize kernels deficient in amylose have a waxy appearance). Waxy-type mutants now exist for many crops including maize, rice, barley, wheat and pea. Varieties of potato and cassava have also been discovered but breeding such traits into elite varieties of these crops is more difficult. Therefore transgenic approaches have also been use to achieve the same impact (e.g. the above-mentioned genetically modified potato plants "Amflora" from BASF).

Given that GBSS is presently the only known target for modifying the amylose content in plants, there is still a need to provide alternative means and methods for providing plants with a modified amylose content.

This need is addressed in the present invention, by providing the embodiments as defined in the claims.

Accordingly, the present invention relates in a first aspect to a method for preparing a plant cell which synthesizes a starch with a reduced amylose content said method comprising the step of genetically modifying a plant cell so that it shows a reduced expression of a PTST protein in comparison to a non-genetically modified plant cell. The present invention therefore relates also to a method for reducing the amylose content of a starch produced in a plant cell said method comprising the step of genetically modifying a plant cell so that it shows a reduced expression of a PTST protein in comparison to a non-genetically modified plant cell.

The present invention is based on the finding that a protein hereinafter referred to as PTST (for Protein Transporting to Starch) has an influence on the synthesis of amylose in plant starch and that a reduction of the amount of said protein or the non-functionality of said protein is correlated with a reduction in the amount of amylose in the starch produced in plants. The results obtained and described in the appended Examples support the notion that the PTST protein is involved in binding GBSS in the stroma and transporting it to the starch granule, where it is released again. A modification of the amount of this protein in the plant cells or rendering the protein non-functional therefore influences the amount of GBSS delivered to the starch granule and consequently the amount of amylose produced in the plant cells.
The term "PTST protein" when used in the scope of the present invention refers to a protein characterized by the following features:
(a) It shows an amino acid sequence as shown in SEQ ID NO:2 or a sequence which is at least 32% identical to the sequence shown in SEQ ID NO:2.
(b) It is targeted to the chloroplasts.
(c) It has an N-terminal domain featuring a coiled-coil.
(d) It has a Carbohydrate-Binding Module (CBM) for interacting with carbohydrates located at the C-terminus.
(e) It can bind to starch.
(f) It can interact with GBSS (Granule Bound Starch Synthase).

The amino acid sequence shown in SEQ ID NO:2 is the amino acid sequence of the PTST protein of *Arabidopsis thaliana.* This protein is encoded by the gene at locus At5g39790 of the *A. thaliana* genome. The coding region is shown in SEQ ID NO:1. The protein is described in Lohmeier-Vogel et al. (2008, BMC Plant Biol. 8, 120) and in the master thesis of Adrianus Petrus Claassens (Stellenbosch University; Faculty of Natural Sciences, Department of Genetics; Institute for Plant Biotechnology; (December 2013)). Lohmeier-Vogel et al. (loc.cit.) report on the structure of the protein and a possible involvement in starch metabolism in plant cells. Claassens (2013) carried out further investigations on a possible role of the PTST protein (referred therein as SP1) in starch metabolism but could not establish an involvement of the protein in the starch metabolism. It was found by Claassens (2013) that the protein has no influence on the overall starch content. Moreover, Claassens (2013) hypothesized that, since the protein contains an AMP-activated kinase domain, it may be involved in starch phosphorylation. However, no such link could be established.
The term "PTST protein" does not only relate to the respective protein from A. *thaliana* but also to PTST proteins from other plants or to variants of the naturally occurring PTST proteins. These naturally occurring PTST proteins were identified through database searches using the Arabidopsis PTST protein sequence. Such proteins show an amino acid sequence which shows at least x% sequence identity to the sequence shown in SEQ ID NO:2, wherein x is selected from the group consisting of 32, 35, 40, 45, 50, 55, 60, 65, 70, 75, 80, 81, 82, 83, 84 and 85. In a preferred embodiment the PTST protein has at least 90% sequence identity, preferably at least 91 %, more preferably at least 92%, even more preferably, at least 93, 94, 95, 96, 97, 98 or 99% identity to the amino acid sequence shown in SEQ ID NO: 2.
Even more preferably, a PTST protein also shows a minimal sequence similarity to the amino acid sequence shown in SEQ ID NO: 2, i.e. at least y% sequence similarity, wherein y is selected from the group consisting of 53, 55, 60, 65, 70, 75, 80, 81, 82, 83, 84 and 85. In a preferred embodiment the PTST protein has at least 90% sequence similarity, preferably at least 91 %, more preferably at least 92%, even more preferably, at least 93, 94, 95, 96, 97, 98 or 99% similarity to the amino acid sequence shown in SEQ ID NO: 2.
The percent of sequence identity or sequence similarity can be determined by different methods and by means of software programs known to one of skill in the art, such as for example the CLUSTAL method or BLAST and derived software, or by using a sequence comparison algorithm such as that described by Needleman and Wunsch (J. Mol. Biol., 1970, 48:443) or Smith and Waterman (J. Mol. Biol., 1981, 147:195). In a preferred embodiment the level of sequence identity or sequence similarity to the Arabidopsis PTST protein is dermined by making use of the BLAST2 program (Tatusova and Madden, 1999, FEMS Microbiol. Lett. 15:247) with default parameters (expect threshold - 10; word size - 3; BLOSUM62 matrix with conditional compositional score matrix adjustment and gap costs of 11 and 1 for existence and extension respectively). Using this algorithm, the most divergent sequence found was that of *Chlamydomonas reinhardtii* (XP_001694419.1) with 32% identity and 53% similarity.
The PTST protein from *A. thaliana* has a predicted molecular weight of about 27 kD. As reported by Lohmeier-Vogel et al. (loc. cit.; see Figure 1 of said document) orthologues to the *A. thaliana* PTST protein can be found in a variety of plants, e.g. in *Helianthus ciliaris, Raphanus raphanistrum, Glycine max, Populus trichocarpa, Citrus clementina, Citrus sinensis, Taraxacum oficinale, Oryza sativa, Hordeum vulgare, Gossypium raimondii, Medicago truncatula, Picea glauca, Pinus taeda* and *Chlamydomonas reinhardtii.*

BLAST scoring reveals orthologs in other plant species such as *Picea sitchensis, Amborella trichocarpa, Brachypodium distachyon, Triticum urartu, Aegilops tauschii, Zea may, Setaria italic, Solanum lycopersicum, Genlisea aurea, Cucumis sativus, Vitis vinifera, Theobroma cacao, Ricinus communis,* Platanus acerifolia, *Prunus persica, Fragaria vesca* subsp. vesca, *Capsella rubella, Arabidopsis lyrata* subsp. lyrata, *Cicer arietinum, Physcomitrella patens* subsp. patens.

As reported in Lohmeier-Vogel et al. (loc.cit.) the different PTST proteins of different plants show a high degree of conservation, in particular in the C-terminal Carbohydrate-Binding-Module (CBM) and in the coiled-coil domain (see below).

The PTST protein is furthermore characterized in that it is targeted to the chloroplasts. Whether a protein is targeted to the chloroplasts can be tested for by method known to the person skilled in the art and are, e.g., described in Lohmeier-Vogel et al. (loc. cit.). One possibility is to isolate chloroplasts from the plant and probe them with antibodies raised against the PTST protein for the presence of the protein. Another possibility is to use a construct encoding the PTST protein, which is fused at its C-terminus with a detectable tag such as a fluorescent protein, e.g., Green Fluorescent Protein (GFP) and to transiently transform cells with this construct and to detect the localization of the protein. For this purpose, one can, e.g., use onion epidermal cells and bombard them with the respective construct using biolistic transformation (see Lohmeier-Vogel et al., loc. cit.), or use transient *Agrobacterium tumefaciens-mediated* transformation of tobacco leaves (see appended Examples). A further possibility is to generate stable transformants expressing PTST protein that is fused at its C-terminus with a detectable tag such as a fluorescent protein, e.g., Green Fluorescent Protein (GFP), and to determine the localization of the protein in the tissue of the transgenic plants.
Proteins that are targeted to the chloroplasts usually possess a chloroplast transit peptide. Methods for analyzing sequences of plant proteins for the presence of a chloroplast transit peptide are well known in the art and are described, e.g., in Lohmeier-Vogel (loc. cit.). Thus, available methods are, e.g., iPSORT (Bannai et al., 2002, Bioinformatics 18, 298-305) PCLR (Schein et al., 2001, Nucleic Acids Res. 29, E82), PREDOTAR (Small et al., 2004, Proteomics 4, 1581-1590) and TARGETP (Emanuelsson et al., 2000, J Mol Biol. 300, 1005-1016). IPSORT utilizes amino acid properties and patterns in a succession of decision nodes to make predictions; PCLR analyses the abundance of different amino acid types, using principle-component analysis and logistic regression; while both PREDOTAR and TARGETP are artificial neural network methods.

As indicated above, a PTST protein is also characterized in that it has an N-terminal domain featuring a coiled-coil. Coiled-coils are structural motifs that occur in many proteins, and often act as platforms for protein-protein interactions (Mason and Arndt, 2005). These interactions are typically mediated through electrostatic and hydrophobic interactions between two α-helices. This domain is highly conserved among the different orthologues of PTST proteins from different plant species. As reported in Lohmeier-Vogel et al. (loc. cit.), this domain is located in the *A. thaliana* PTST protein from around residues 90 to 195 (with a strongly predicted region ranging from residues 90 to 150 and with a more weakly predicted region ranging from residues 160 to 195). Means and methods for analyzing amino acid sequences of proteins to predict the presence of coiled-coils are well-known in the art and are described, e.g. in Lohmeier-Vogel et al. (loc. cit.), for example PairCoil2 (McDonnell et al., 2006, Bioinformatics. 22, 356-358), PCOILS (Gruber et al., 2006, J. Struct. Biol. 155, 140-145) and Marcoil (Delorenzi and Speed, 2002. Bioinformatics. 18, 617-625). PairCoil2 and PCOILS utilize a Position Specific Scoring Matrix (PSSM) approach in combination with a fixed-length sliding window (28 or 21 residues). The former technique is improved by using pairwise residue probabilities, the latter by using profile-profile comparisons. Marcoil reports predicted CC sequence regions under various threshold probabilities for a true positive result (e.g. 10%, 50%, or 90% threshold). PCOILS presents a graphical output with predicted CC regions depicted against a vertical axis representing the probability for a true positive result (e.g. 0.90). PairCoil2 presents both a graphical and numerical output. Here the default threshold for a region being predicted as a coiled-coil is that the probability of a false positive result be less than 0.025.
It is possible to identify a conserved region in the coiled-coil between the different PTST proteins. In the PTST protein from *Arabidopsis thaliana* (SEQ ID NO: 2) this conserved region is located between amino acid residues 101-185 (LVKKLSEANQQNRFLKRQEHEITNIKTELALMELEVQALVKLAEEIANLGIPQGSRKI SGKYIQSHLLSRLDAVQKKMKEQIKGV; SEQ ID NO: 3). In a preferred embodiment, a PTST protein shows in its coiled-coil no less than x% sequence identity to the region of residues 101-185 of SEQ ID NO: 2 when aligned to SEQ ID NO: 2, with x being selected from the group consisting of 28, 30, 35, 40, 45, 50, 55, 60, 65, 70, 75, 80, 81, 82, 83, 84 and 85. In a preferred embodiment the PTST protein shows in its coiled-coil at least 90% sequence identity, preferably at least 91 %, more preferably at least 92%, even more preferably, at least 93, 94, 95, 96, 97, 98 or 99% identity to the amino acid sequence shown in SEQ ID NO: 2 in the range of residues 101-185 of SEQ ID NO: 2.
Even more preferably, a PTST protein also shows in its coiled-coil a minimal sequence similarity to the amino acid sequence of residues 101 to 185 of SEQ ID NO: 2, i.e. at least y% sequence similarity, wherein y is selected from the group consisting of 48, 50, 55, 60, 65, 70, 75, 80, 81, 82, 83, 84 and 85. In a preferred embodiment the PTST protein shows in its coiled-coil at least 90% sequence similarity, preferably at least 91 %, more preferably at least 92%, even more preferably, at least 93, 94, 95, 96, 97, 98 or 99% similarity to the amino acid sequence shown in SEQ ID NO: 2 in the range of residues 101-185 of SEQ ID NO: 2. As regards the determination of the sequence identity or sequence similarity the same applies as has been set forth above.

As indicated above, the PTST protein is also characterized by the fact that it has a Carbohydrate-Binding Module (CBM) for interacting with carbohydrates located at the C-terminus. CBMs describe conserved non-catalytic binding domains that are commonly found in polysaccharide-binding proteins. Conserved CBMs are classified into families based on amino acid sequence curated by the Carbohydrate Active Enzymes database (CAZy; http://www.cazy.org). CBMs are typically modular domains that can fold and function autonomously (Cantarel et al. 2009, Nucleic Acids Res. 37 (Database issue), D233-D238).
Preferably, the CBM present in the PTST protein falls into the CBM48 class. CBM48s are modules of approximately 100 residues with glycogen-binding function, and are typically found on GH13 modules. It is also found on the beta-subunit of AMP-activated protein kinases. CBM48s can be found in many other starch-active enzymes, in particular the starch branching and debranching enzymes (e.g. isoamylases and limit dextrinases), as well as the glucan phosphatases STARCH EXCESS 4 and LIKE SEX FOUR1, where they mediate binding to starch.

In PTST, this domain is located at the C-terminus of the protein, and is very highly conserved among PTST proteins from different plant species (see Lohmeier-Vogel (loc. cit.), Figure 1). In the sequence of the PTST protein of *A. thaliana* the CBM is located in the region ranging from amino acid residues 191-272 (KEVHVFWIGMAESVQVMGSFDGWSQREDLSPEYSALFTKFSTTLFLRPGRYEMKF LVDGEWQISPEFPTSGEGLMENNVLVV; SEQ ID NO: 4). In a preferred embodiment, a PTST protein shows in its CBM no less than x% sequence identity to the region of residues 191-272 of SEQ ID NO:2 when aligned to SEQ ID NO:2, with x being selected from the group consisting of 41, 45, 50, 55, 60, 65, 70, 75, 80, 81, 82, 83, 84 and 85. In a preferred embodiment the PTST protein shows in its CBM at least 90% sequence identity, preferably at least 91 %, more preferably at least 92%, even more preferably, at least 93, 94, 95, 96, 97, 98 or 99% identity to the amino acid sequence shown in SEQ ID NO: 2 in the range of residues 191-272 of SEQ ID NO: 2. Even more preferably, a PTST protein also shows in its CBM a minimal sequence similarity to the amino acid sequence of residues 191-272 of SEQ ID NO: 2, i.e. at least y% sequence similarity, wherein y is selected from the group consisting of 62, 65, 70, 75, 80, 81, 82, 83, 84 and 85. In a preferred embodiment the PTST protein shows in its CBM at least 90% sequence similarity, preferably at least 91%, more preferably at least 92%, even more preferably, at least 93, 94, 95, 96, 97, 98 or 99% similarity to the amino acid sequence shown in SEQ ID NO: 2 in the range of residues 191-272 of SEQ ID NO: 2.
As regards the determination of the sequence identity or sequence similarity the same applies as has been set forth above.

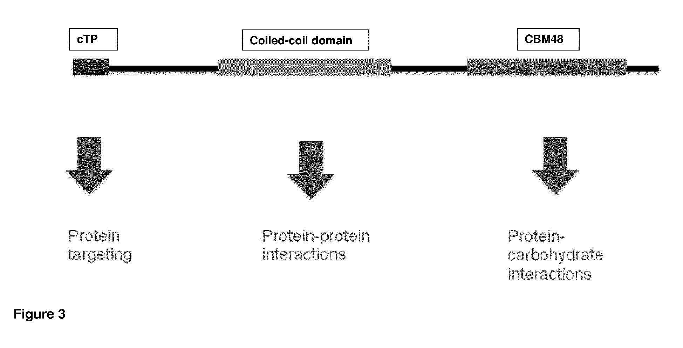

The overall structure of the PTST protein is shown in Figure 3. From this figure, it is evident that the PTST protein has a unique structure in that it has a chloroplast transit peptide at the N-terminus, a coiled-coil (CC) domain and a Carbohydrate-Binding Module (CBM) at the C-terminus.

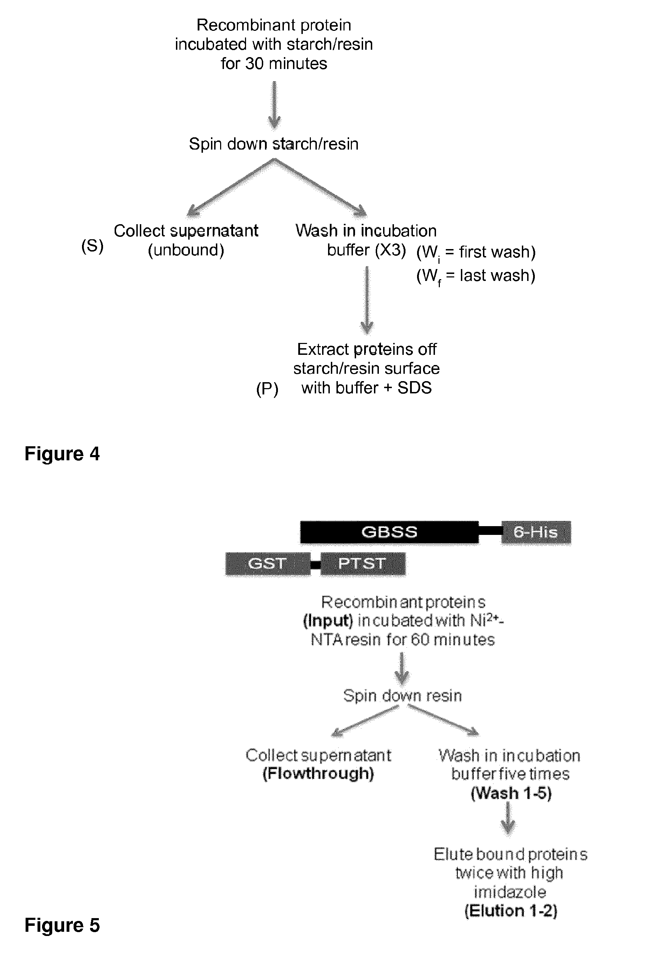

A PTST protein is further characterized by the feature that it can bind to starch. The binding of starch can be assessed by methods known to the person skilled in the art and as described, e.g., in Lohmeier-Vogel (loc. cit.; "Polysaccharide binding assays"). In particular, such an assay may be carried out as follows and as outlined in Figure 4: the protein (e.g. a recombinantly produced protein) and the starch (or a non-glucan resin such as Sephadex as a control) are incubated together for 30 minutes. The starch/resin is spun down by centrifugation. The supernatant (S) is collected. The pellet is washed in incubation buffer, re-pelleted by centrifugation, and the wash fraction (the supernatant) is collected. This wash step is repeated twice, to make a total of three washes. The pellet is treated with buffer + SDS to extract the proteins off the starch/resin surface, which leads to fraction P. Both the S and P fractions, along with the first and last washes (Wᵢ and W_{f} respectively) are loaded on an SDS-PAGE for analysis. Alternatively, such an assay can be carried out as described by Loheimer-Vogel et al. (loc. cit.): Starch is dissolved in PBS, pH 7.3. A mixture of protein (e.g. 1µg/ml) and starch (e.g. 10 mg/ml) is incubated in a microfuge tube for 1 h at room temperature with end over end mixing in PBS. The solution is centrifuged for 30s at 500 rpm and the supernatant is carefully transferred into a new microfuge tube. The pellets are washed 5 times, each time with resuspension in fresh PBS, gentle mixing, centrifugation and removal of the supernatant. After the final wash the pellets are transferred into a fresh microfuge tube and mixed with an equal volume of 2xSDS-PAGE cocktail. The samples are processed for 5 min at 95°C and loaded onto an SDS-PAGE.
Preferably, starch binding is assessed by an assay as described in the appended Examples.
In a preferred embodiment, the PTST protein binds to native starch (i.e. starch containing amylose as well as amylopectin) as well as to amylose-free starch or to amylose.
As is evident from the appended Examples, it could be shown that the interaction of the PTST protein with polysaccharides such as starch takes place via the CBM domain.

A PTST protein is further characterized by the feature that it can interact with GBSS. As is shown in the appended Examples, a PTST protein is able to bind to GBSS, in particular through its coiled-coil domain. Such an interaction, i.e. binding, can be assessed by methods well-known to the person skilled in the art, such as pull-down assays. An example of a pull-down assay is schematically shown in Figure 5. In this example the GBSS protein contains a His-tag and the PTST contains a GST (glutathione-S-transferase)-tag. The proteins are incubated with Ni²⁺-NTA resin, which interacts only with His-tagged proteins. The resin is spun down and the supernatant is collected. The pellet is washed with incubation buffer 5 times. Subsequently, the proteins bound to the resin are eluted twice with high imidazole and the eluted proteins are analyzed, e.g. in SDS-PAGE. The presence of PTST in the eluted fraction indicates that it can interact with GBSS. The corresponding assay is also described in detail in the appended Examples. In a preferred embodiment, the PTST protein is able to bind to a GBSS protein from the same plant species.
GBSS proteins are starch synthases which transfer an activated glucose residue (e.g. ADP-glucose) to a (1,4-α-D-glucosyl)ₙ molecule so as to lead to (1,4-α-D-glucosyl)ₙ₊₁. GBSS proteins are distinguished from other starch synthases (SSI, SSII, SSIII, SSIV) by their localisation within the starch granule matrix. GBSS proteins are responsible for the synthesis of amylose. There are two major classes of GBSS proteins (Cheng et al. 2012, PLoS ONE 7, e30088). Proteins from the GBSSI class are exclusively found in cereal crop species (ie: rice, wheat, barley, etc), and are found exclusively in the endosperm of grains. In cereals, GBSSII class proteins are found primarily in leaf tissues. Non-cereal plant species only contain GBSSII class proteins. In a preferred embodiment, the PTST protein can bind to both forms of GBSS.
GBSS proteins share a considerable degree of sequence identity. Cheng et al. (loc. cit.) report that GBSSI and GBSSII genes in monocots share about 63% sequence identity on the nucleotide level. GBSSI genes in monocots and GBSSII genes in eudicots are said to share between 59% and 67% sequence identity in the coding regions. Moreover, the coding sequences of GBSSII genes in monocots are described as showing 67% to 70% sequence identity to the GBSS genes in eudicots.

As mentioned above, the plant cell prepared according to the method of the present invention is genetically modified so as to show a reduced expression of a PTST protein in comparison to a corresponding non-genetically modified plant cell and synthesizes a starch with a reduced amylose content in comparison to a corresponding non-genetically modified plant. The term "a starch with a reduced amylose content" means that the genetically modified plant cell according to the invention synthesizes starch that shows a reduced amylose content when compared to starch synthesized in a corresponding non-genetically modified plant cell. The term "reduced amylose content" means that the amylose content in the starch produced in a genetically modified plant cell according to the invention is not more than 95%, preferably not more than 90%, more preferably not more than 80% even more preferably not more than 70%, 60%, 50%, 40%, 30%, 20% or 10% of the amylose content in the starch produced by a corresponding not genetically modified plant cell. Most preferably the starch synthesized by a plant cell prepared according to the method of the present invention is free of amylose.
The term "a corresponding plant cell which is not genetically modified" in particular refers to a plant cell which was used as a starting material for the preparation of the genetically modified plant cell according to the present invention, i.e. cells which have the same genetic information as the genetically modified plant cells according to the invention except for the genetic modification effected in the genetically modified cells as described herein. Generally, a corresponding non-genetically modified plant is a corresponding wild-type plant. The reduction in the expression of a PTST protein means that the genetically modified plant shows a reduced amount of the PTST protein in comparison to a corresponding non-genetically modified plant. A reduced amount of the PTST protein means a reduction in the amount of the protein of at least 20%, more preferably of at least 30%, at least 40%, at least 50%, at least 60%, at least 70%, at least 80%, even more of at least 90% and particularly preferred of 100% when compared to a corresponding non-genetically modified plant. Thus, in a particularly preferred embodiment, no PTST protein is present in a plant prepared according to a method of the present invention.
The amount of a PTST protein can be determined by methods known to the person skilled in the art, e.g. by immunological methods such as Western Blot, or by quantitative mass-spectrometry-based proteomic approaches.
A reduced amount of a PTST protein can also mean a reduced amount of a functional PTST protein. A functional PTST protein is understood to be a PTST protein that has the capacity to be translocated into the chloroplasts, which can bind to starch and which can bind to GBSS. The amount of functional PTST protein in a plant can be assessed by the assays described herein above in connection with the description of the properties of a PTST protein.
In an alternative embodiment, a reduced amount of the PTST protein means a reduction in the amount of the transcripts encoding a PTST protein in cells of a plant. A reduced amount of transcripts encoding a PTST protein means a reduction in the amount of transcripts encoding a PTST in photosynthetically active cells of at least 20%, more preferably of at least 30%, at least 40%, at least 50%, at least 60%, at least 70%, at least 80%, even more of at least 90% and particularly preferred of 100% when compared to a corresponding non-genetically modified plant. Thus, in a particularly preferred embodiment, no transcripts encoding a PTST protein are present in a plant cell prepared according to a method of the present invention. Such transcripts can be detected by methods well-known to the person skilled in the art such as Northern Blot analysis, microarray analysis, or quantitative RNA-sequencing or RT-PCR approaches.

The reduction of the expression of the PTST protein in a plant cell can be achieved by methods known to the person skilled in the art. One possibility is, e.g., to prepare genetically modified plant cells in which the gene encoding the PTST protein is completely deleted or in which it is disrupted or mutated so as to render the gene non-functional. "Non-functional" preferably means that the gene does no longer lead to the production of a protein or of a functional protein. A non-functional PTST protein is in particular a PTST protein that is no longer capable of being translocated into the chloroplast and/or is no longer capable of binding to starch and/or to GBSS. Corresponding methods for deleting, disrupting or mutating a gene in plant cells are well-known in the art and are described. One possibility for disrupting a gene so as to render it non-functional is mutagenesis via T-DNA insertion (Krysan et al. 1999, Plant Cell 11, 2283-2290; Jeon et al. 2000, Plant J. 22, 561-570). Another non-transgenic means to reduce the expression of the PTST protein in a plant cell is the use of chemical mutagens, such as EMS, to introduce point mutations randomly in the genome (Koornneef et al. 1982, Theor. Appl. Genet. 61, 385-393; Kim et al. 2006, Methods Mol. Biol. 323, Arabidopsis Protocols, 2nd Edition, J. Salinas and J. J. Sanchez-Serrano Eds., Humana Press Inc., Totowa, NJ). Techniques such as 'Targeting Induced Local Lesions In Genomes' (TILLING) may be used to screen for mutations in the PTST gene (McCallum et al. 2000, Plant Physiol. 123, 439-442). Such mutations could cause stop codons that can destabilise the gene transcript and/or lead to the production of a truncated, non-functional protein. Another possibility is that the introduction of mutations within the coding region of the PTST protein will lead to non-functional mutants of the PTST proteins. A non-functional PTST mutant protein is in particular a PTST protein which is no longer capable of being translocated into the chloroplast and/or is no longer capable of binding to starch and/or to GBSS. The appended Examples illustrate such mutants in the form of the W217A/W255A site-directed mutant of the *A. thaliana* PTST protein, in which two key tryptophan residues in the CBM48 domain have been mutated to alanine residues. These mutations lead to a loss of the capacity of the PTST protein to bind to glucans, in particular starch. Means and methods for introducing site-specific mutations into genes are well-known to the person skilled in the art.
A further possibility is the disruption or mutation of the promoter region or in the UTR by any of the above described methods which leads to a reduction of complete abolition of the transcription of the PTST gene and/or translation of the PTST protein. Also these disruptions or mutations in the promoter region or in the UTR are covered by the term "deletion or disruption of an endogenous gene encoding a PTST protein" as used herein.
A further possibility to reduce the expression of the PTST protein in a plant cell is the use of approaches for gene silencing based on RNA, such as the expression of siRNA, RNAi or cosuppression RNA. siRNA, RNAi and cosuppression approaches are well known to the person skilled in the art and are described (e.g. Wesley et al., 2001, Plant J. 27, 581-590; Lu et al., 2003, Methods 30, 296-303; Felippes et al., 2012, Plant J. 70, 541-547). The successful use of such approaches in plants has been reported (e.g. Asatsuma et al., 2005, Plant Cell Physiol. 46, 858-869; Regina et al., 2007, Proc. Natl. Acad. Sci. USA 103, 3546-3551).

Another possibility for the reduction of the expression of the PTST protein is the genetic modification of the plant cell so as to express a corresponding antisense RNA which is complementary to transcripts encoding a PTST protein. Antisense approaches are well-known to the person skilled in the art. In order to cause an antisense-effect during the transcription in plant cells, an antisense molecule should preferably have a length of at least 15 nucleotides, more preferably of at least 50 or 100 nucleotides, even more preferably of at least 500 nucleotides and particularly preferred of at least 750 nucleotides. As is known to the person skilled in the art, an antisense molecule does not have to show 100% sequence identity to the target messenger RNA. It is generally sufficient to show a high degree of sequence identity, preferably at least 85%, more preferably at least 90%, even more preferably at least 95%, 96%, 97% or 98 % and particularly preferred at least 99% sequence identity.

Another possibility for the reduction of the expression of the PTST protein is the genetic modification of the plant cell via the use of genome editing with engineered nucleases. Means and methods for site-specific genome editing whereby DNA is inserted, replaced, or removed from a chosen locus in the plant cell genome are well-known to the person skilled in the art. The engineered site-specific nucleases (e.g. Zinc finger nucleases (ZFNs), Transcription Activator-Like Effector Nucleases (TALENs), the CRISPR/Cas system, and engineered meganuclease re-engineered homing endonucleases) create a specific double-stranded break at the desired locations in the genome, and harness the cell's endogenous mechanisms to repair the induced break by natural processes of homologous recombination (HR) and nonhomologous end-joining (NHEJ). There are currently four families of engineered nucleases being used: Zinc finger nucleases (ZFNs), Transcription Activator-Like Effector Nucleases (TALENs), the CRISPR/Cas system, and re-engineered homing endonucleases. The successful use of such approaches in plants has been reported (e.g. Chen and Gao, 2013, J Genet. Genomics 40, 271-279; Belhaj et al., 2013, Plant Methods 9, 39; Mauricio et al., BMC Biotechnol. 2012, 12:86; Weinthal et al., 2010, Trends Plant Sci. 15, 308-321)

A further possibility to reduce the expression of the PTST protein in a plant cell is the expression of a ribozyme, which is capable of specifically cleaving transcripts encoding a PTST protein. Ribozyme approaches are well-known to the person skilled in the art and have been described, e.g. in Haseloff and Gerlach (1988, Nature 334, 585-591). The successful use of a ribozyme approach in plants has, for example, been described in Borovkov et al. (1996, J Plant Physiol. 147: 644-652) and de Feyter et al. (1996, Mol. Gen. Genet. 250, 329-338).

Thus, the method according to the present invention is preferably a method, wherein the genetic modification of the plant cell, either by transgenic or non-transgenic means, consists of a genetic modification selected from the group consisting of:
(a) deletion or disruption of an endogenous gene encoding a PTST protein;
(b) mutation of an endogenous PTST gene so as to no longer express a PTST protein or so as to encode a non-functional PTST protein;
(c) expression of an siRNA, an RNAi or a cosuppression RNA directed against transcripts encoding a PTST protein;
(d) expression of an antisense RNA which is complementary to transcripts encoding a PTST protein; and
(e) expression of a ribozyme directed against transcripts encoding a PTST protein.

In principle, the method according to the present invention can be applied to any plant cell that synthesizes starch. Preferably the plant cell modified according to the above described method according to the present invention is not an *Arabidopsis thaliana* plant cell, in particular an *A. thaliana* plant cell having a T-DNA insertion in the gene encoding a PTST protein, such as, e.g., *A thaliana* T-DNA insertion mutants SALK_024994.55.50x, SALK_090838.49.15.x, SALK_129717.55.00.x, SALK_025022.56.00.x, SALK_012617 (Alonso et al. 2003, Science, 301, 653-657) and FLAG_622E03 (Samson et al. 2002, Nucleic Acids Res. 30, 94-97).
More preferably, the plant cell is a cell of a starch-storing plant, even more preferably of a starch-storing plant as defined further below.

The present invention also relates to the use of a nucleic acid molecule encoding a PTST protein or of a part of such a nucleic acid molecule for the preparation of a genetically modified plant cell which shows a reduced expression of said PTST protein in comparison to a non-genetically modified plant cell and which synthesizes starch with a reduced amylose content. As mentioned above, the reduction of the expression of a PTST protein can be achieved by using RNA approaches. These approaches make use of molecules that are at least partly homologous to the coding region of a PTST gene or a corresponding mRNA and, thus, involve the use of a nucleic acid molecule encoding a PTST protein or of a part of such a nucleic acid molecule. A part of a nucleic acid molecule encoding a PTST protein refers preferably to nucleic acid molecules having a length of at least 5 nucleotides, more preferably of at least 10 nucleotides, even more preferably of at least 20 nucleotides and particularly preferred of at least 50 nucleotides, at least 100 nucleotides or at least 200 nucleotides. In another preferred embodiment such a part has a length in the range of 5 to 200 nucleotides, preferably in the range of 10 to 100 nucleotide and more preferably in the range of 20 to 50 nucleotides.

The present invention also relates to a genetically modified plant cell which is genetically modified so as to synthesize a starch with a reduced amylose content due to the reduced expression of a PTST protein in comparison to a non-genetically modified plant cell, wherein the genetic modification of the plant consists of a genetic modification selected from the group consisting of:
(a) deletion or disruption of an endogenous gene encoding a PTST protein;
(b) mutation of an endogenous PTST gene so as to encode a non-functional PTST protein;
(c) expression of an siRNA, an RNAi or a cosuppression RNA directed against transcripts encoding a PTST protein;
(d) expression of an antisense RNA which is complementary to transcripts encoding a PTST protein; and
(e) expression of a ribozyme directed against transcripts encoding a PTST protein.
Preferably said genetically modified plant cell is not an *Arabidopsis thaliana* plant cell, in particular an *A. thaliana* plant cell having a T-DNA insertion in the gene encoding a PTST protein, such as, e.g., *A thaliana* T-DNA insertion mutants SALK_024994.55.50x, SALK_090838.49.15.x, SALK_129717.55.00.x, SALK_025022.56.00.x, SALK_012617and FLAG_622E03.
More preferably, the plant cell is a cell of a starch-storing plant, even more preferably of a starch-storing plant as defined further below.

Furthermore, the present invention also relates to a genetically modified plant which contains genetically modified plant cells according to the present invention and which synthesizes a starch with a reduced amylose content as defined herein above. In a preferred embodiment, the genetically modified plant is a starch storing plant. The term "starch storing plant" refers to a plant which produces starch as a storage compound and stores it for long-term storage in special tissues such as in seeds (e.g. in the embryo or the endosperm) or in fruits, tubers, stems, leaves or roots. Such starch-storing plants may be monocotyledonous or dicotyledonous plants. Examples for starch storing plants include rye, barley, wheat, oat, millet, rice, maize, peas, cow peas, wrinkled peas, chickpea, lentils, beans, mung bean, cassava, arrowroot, sweet potato, yarn, taro, potato, tomato, banana, sago and a starch storing alga (e.g. *Chlamydomonas reinhardtii).*
In a particularly preferred embodiment the genetically modified plant cell according to the present invention is a cell of a starch storing tissue of a starch storing plant, preferably a tuber cell, an endosperm cell or a root cell.

The present invention also relates to parts of the genetically modified plants according to the present invention which produce a starch with a reduced amylose content, said parts containing genetically modified plant cells according to the invention as described herein-above. Such parts include, e.g., harvestable parts, preferably starch-storing organs such as tubers, grains, leaves, stems or roots.
The present invention also relates to propagation material of a genetically modified plant according to the invention containing genetically modified cells according to the invention. Such propagation material includes, e.g. seeds, root stocks, tubers, cuttings and fruits.

The present invention also relates to a method for producing a starch with a reduced amylose content comprising the step of extracting starch from a plant according to the present invention, containing plant cells according to the present invention synthesizing a starch with a reduced amylose content or from parts of such a plant. Such a method may also further comprise the step of harvesting a plant according to the present invention containing plant cells according to the present invention synthesizing a starch with a reduced amylose content or harvesting parts of such a plant. It may also further comprise the step of cultivating a plant according to the present invention containing plant cells according to the present invention synthesizing a starch with a reduced amylose content.

The present invention also relates to a method for producing in a plant a starch with a reduced amylose content comprising the steps of
(i) reducing in said plant the amount of a PTST protein produced by cells of said plant; and
(ii) obtaining starch from said plant.
In principle such a method according to the present invention can be applied to any plant cell which synthesizes starch. Preferably the plant cell modified according to the above described method according to the present invention is not an *Arabidopsis thaliana* plant cell, in particular an *A. thaliana* plant cell having a T-DNA insertion in the gene encoding a PTST protein, such as, e.g., *A thaliana* T-DNA insertion mutants SALK_024994.55.50x, SALK_090838.49.15.x, SALK_129717.55.00.x, SALK_025022.56.00.x, SALK_012617and FLAG_622E03.
More preferably, the plant cell is a cell of a starch-storing plant, even more preferably of a starch-storing plant as defined further below.

The skilled person is familiar with methods for extracting the starch of plants or of starch-storing parts of plants. Furthermore, methods for extracting the starch from various starch-storing plants have been described, e.g. in "Starch: Chemistry and Technology (ed.: Whistler, BeMiller and Paschall (1994), 2ndedition, Academic Press Inc. London Ltd.; ISBN 0-12-746270-8; cf. e.g. chapter XII, page 412-468: Maize and Sorghum Starches: Production; by Watson; chapter XIII, page 469-479; Tapioca, Arrow Root and Sago Starches: Production; by Corbishley and Miller; chapter XIV, page 479-490: Potato Starch: Production and Applications; by Mitch; chapter XV, page 491 to 506: Wheat Starch: Production, Modification and Applications; by Knight and Oson; and chapter XVI, page 507-528: Rice Starch: Production and Applications; by Rohmer and Klem; Maize Starch: Eckhoff et al., (1996), Cereal Chem. 73 54-57, the extraction of maize starch on an industrial scale is usually achieved by means of the so-called wet milling). Appliances that are usually used for methods for extracting starch from plant material include separators, decanters, hydrocyclones, spray dryers and fluid-bed dryers.

The starch produced by a method according to the present invention as described herein above, is also an object of the present invention.

The present invention also relates to a genetically modified plant cell that produces a starch with an increased amylose content in comparison to a corresponding non-genetically modified plant cell and which is characterized in that it is genetically modified by the introduction of a nucleic acid molecule which encodes a PTST protein and which is expressed in said plant cell.

In principle the genetically modified plant cell may be a plant cell of any possible plant species. It may be a plant cell of a monocotyledonous or of a dicotyledonous plant. Preferably the plant cell is of a cultivated plant, such as a vegetable plant (e.g. tomatoes) or of a starch storing plant such as those mentioned further below. It is also conceivable that the plant cell is of another useful plant, e.g. oil-producing plants, such as oils seed rape or sunflower, or of hemp, flax or pastures like white clover, ryegrass or alfalfa.
In one preferred embodiment said genetically modified plant cell is not an onion epithelial cell or a cell of *A. thaliana.*

In another preferred embodiment said genetically modified plant cell is a cell of a starch storing plant. The term "starch storing plant" refers to a plant which produces starch as a storage compound and stores it for long-term storage in special tissues such as in seeds (e.g. in the endosperm or embryo), or in tubers, stems, leaves or roots. Such starch-storing plants may be monocotyledonous or dicotyledonous plants. Examples for starch storing plants include rye, barley, wheat, oat, millet, rice, maize, peas, cow peas, wrinkled peas, chickpea, lentils, beans, mung bean, cassava, arrowroot, sweet potato, yarn, taro, potato, tomato, banana, sago and a starch storing alga (e.g. *Chlamydomonas reinhardtii).*
In a particularly preferred embodiment the plant cell is a cell of a starch storing tissue of a starch storing plant, preferably a tuber cell, an endosperm cell or a root cell.

In another preferred embodiment, the genetically modified plant cell according to the present invention is genetically modified by the introduction of a nucleic acid molecule encoding a PTST protein which is expressed in said plant cell, wherein said nucleic acid molecule is not a nucleic acid molecule coding for a C-terminal GFP (Green Fluorescent Protein) fusion construct of the *A. thaliana* PTST protein, preferably the C-terminal GFP fusion construct of the *A. thaliana* PTST protein as described in Lohmeier-Vogel et al. (loc. cit.), even more preferably the GFP fusion construct contained in the vector At5g39790-pK7FWG2. At5g39790 is the designation used in said publication for the PTST protein of *A. thaliana.* In this construct the At5g39790 gene sequence (inclusive the chloroplast targeting sequence) was engineered to introduce *attB1* and *attB2* recombination sites at the N-and C-terminal regions of the gene by using the primers as described in the section "Cloning of the At5g39790 gene products" and recombined into the entry vector pDonor201 using the Gateway BP clonase II enzyme mix. The resulting plasmids were then transformed into *E. coli* DH5α, selected for kanamycin resistance before being recombined into the Gateway vector pK7FWG2 (Trends Plant Sci. 7 (2002), 193-195) upstream of the GFP using the LR Clonase II enzyme mix. In the context of the present invention, a plant which is genetically modified by introduction of a nucleic acid molecule is referred to as "transgenic".

The term "a starch with an increased amylose content" means that the genetically modified plant cell according to the invention synthesizes a starch which shows an increased amylose content when compared to starch synthesized in a corresponding non-genetically modified plant cell, i.e. a corresponding plant cell which is not genetically modified by the introduction of a nucleic acid molecule encoding a PTST protein which is expressed in said plant cell. The term "increased amylose content" means that the percentage amylose content of the starch produced in a genetically modified plant cell according to the invention is at least 5%, preferably at least 7% higher, more preferably at least 10% higher, even more preferably at least 20% higher and particularly preferred at least 30% higher than the amylose content of the starch produced by a corresponding non-genetically modified plant cell. This means, e.g., that if the percentage amylose content is 10% higher in the genetically modified plant cell in comparison to the non-genetically modified plant cell and the non-genetically modified plant cell has an amylose content of 30%, then the genetically modified plant cells show an amylose content of 40%.
The term "a corresponding plant cell which is not genetically modified" in particular refers to a plant cell which was used as a starting material for the preparation of the genetically modified plant according to the present invention, i.e. cells which have the same genetic information as the genetically modified plants according to the invention except for the genetic modification effected in the genetically modified cell, preferably the introduction of a nucleic acid encoding a PTST protein.

In a preferred embodiment a genetically modified plant cell according to the invention is also characterized in showing an increased amount of a PTST protein in comparison to a corresponding non-genetically modified plant cell, i.e. a corresponding plant which is not genetically modified by the introduction of a nucleic acid molecule encoding a PTST protein which is expressed in said plant cell. The term "an increased amount of a PTST protein" means that in the genetically modified plant cell according to the present invention the amount of a PTST protein is increased in comparison to a corresponding non-genetically modified plant cell. Such an increase can be detected by methods known to the person skilled in the art, e.g. immunological methods such as Western Blot or quantitative mass-spectrometry-based proteomic approaches. The term "increased amount" preferably means that the amount of the PTST protein is increased by at least 10%, more preferably by at least 20%, even more preferably by at least 50% and particularly preferred by at least 100% when compared to a corresponding non-genetically modified plant cell.

In a further preferred embodiment, a genetically modified plant cell according to the invention is also characterized in showing an increased amount of transcripts encoding a PTST protein in comparison to a corresponding non-genetically modified plant cell, i.e. a corresponding plant which is not genetically modified by the introduction of a nucleic acid molecule encoding a PTST protein which is expressed in said plant cell. The term "an increased amount of transcripts encoding a PTST protein" means that in the genetically modified plant cell according to the present invention, the amount of transcripts encoding a PTST protein is increased in comparison to a corresponding non-genetically modified plant cell. Such an increase can be detected by methods known to the person skilled in the art, e.g. by Northern Blot, microarray analysis, or quantitative RNA-sequencing or RT-PCR approaches. The term "increased amount" preferably means that the amount of transcripts encoding the PTST protein is increased by at least 10%, more preferably by at least 20%, even more preferably by at least 50% and particularly preferred by at least 100% when compared to a corresponding non-genetically modified plant cell.

The genetically modified plant cell according to the present invention is preferably characterized in that it is transformed with a nucleic acid molecule encoding a PTST protein that provides for its expression in plant cells. Such a plant is also referred to as a transgenic plant. The term "transformed" means that the nucleic acid encoding a PTST protein is stably introduced in to the genome of the genetically modified plant cell and is controlled by a promoter active in plant cells, in particular in plant cells in which it is desired that the PTST protein is to be expressed.
The genetically modified plant cells according to the present invention differ from naturally occurring plant cells, in particular from the corresponding non-genetically modified cells, among other things in that at least one copy of the nucleic acid encoding a PTST protein is integrated into their genome of the genetically modified plant, possibly in addition to naturally occurring copies. Preferably this/these additional copy/copies is/are integrated at a location in the genome of the plant cell at which the possibly existing endogenous gene encoding a PTST protein does not naturally occur. This may be determined, e.g., by Southern Blot analysis.

In a preferred embodiment, the nucleic acid molecule encoding a PTST protein that is introduced into the plant cells and stably integrated in its genome is foreign with respect to the plant cell. The term "foreign" means that such a nucleic acid molecule does not naturally occur in the plant cell from which the genetically modified cell according to the invention is derived by the introduction of the nucleic acid molecule. If the PTST protein that is encoded by the nucleic acid molecule is homologous to the plant cell, i.e. does naturally occur in the plant cell, the term "foreign" means that the nucleic acid molecule contains at least one component that does not naturally occur in said plant cell. Preferably said component is a regulatory element for expression in plant cells, preferably a promoter, which is naturally not part of the PTST gene of the plant cell into which the nucleic acid molecule is introduced. In other words, it is a regulatory element for expression in plant cells that is heterologous with respect to the nucleotide sequence encoding the PTST protein to be introduced into the plant cell.
As mentioned above, a PTST protein is characterized in that it is translocated into the chloroplast. As such, it normally has a chloroplast transit peptide which ensures localization of the protein in the plastids. In one embodiment, the nucleic acid molecule that is introduced into the plant cells encodes a PTST protein which includes its natural chloroplast transit peptide. In another embodiment, the nucleic acid that is introduced into the plant cells encodes a PTST protein in which the naturally occurring chloroplast transit peptide is replaced by a peptide from another protein that ensures the translocation of the protein into the plastids, in particular the chloroplasts, e.g. a PTST protein from another species or from a different protein. An example for such a peptide is the transit peptide of the plastidic Ferredoxin: NADP⁺ oxidoreductase (FNR), e.g. of the FNR of spinach (Jansen et al., 1988, Current Genet. 13, 517-522). In particular, the sequence ranging from nucleotides -171 to 165 of the cDNA sequence disclosed therein can be used, which comprises the 5' untranslated region as well as the sequence encoding the transit peptide. A further example is the transit peptide of the waxy protein, e.g. of the waxy protein of maize (Klösgen et al., 1989, Mol. Gen. Genet. 217, 155-161), or the transit peptide of the ribulose bisphosphate carboxylase small subunit (see, e.g., Wolter et al., 1988, Proc. Natl. Acad. Sci. USA 85, 846-850 and Nawrath et al., 1994, Proc. Natl. Acad. Sci. USA 85, 12760-12764), of the NADP malate dehydrogenase (see, e.g., Gallardo et al., 1995, Planta 197, 324-332) or of the glutathione reductase (see, e.g., Creissen et al., 1995, Plant J. 8, 167-175).

The nucleic acid molecule that is introduced into the plant cells according to the invention comprises regulatory elements that allow for the expression of the encoded PTST protein in plant cells. Such regulatory elements are known to the person skilled in the art and comprise, e.g. promoters and enhancers and terminators. There is a plurality of promoters or regulatory elements at disposal for expressing a nucleic acid molecule of the invention in plant cells. In principle, all promoters, enhancers, terminators, etc. that are active in plants are regulatory elements for the expression in plant cells. Basically any promoter that is functional in the plants selected for the expression of the PTST protein can be used. With regard to the plant species used, the promoter can be homologous or heterologous. Said promoter may be selected in such a way that the expression takes place in a constitutive manner or only in a particular tissue, at a certain time in the development of the plant, or at a time that is determined by external influence. Examples of suitable promoters are the 35S promoter of the cauliflower mosaic virus (Odell et al., 1985, Nature 313, 810-812 or US 5 352 605), which ensures a constitutive expression in all tissues of a plant, and the promoter construct described in WO/9401571. The ubiquitin promoter (see. e.g. US 5 614 399) and the promoters of the polyubiquitin genes from maize are further examples. However, also promoters that are only activated at a time determined by external influence (inducible promoters - see e.g. WO/9307279) can be used. Promoters of heat shock proteins allowing a simple induction may be of particular interest. Furthermore, promoters that lead to the expression of downstream sequences in a certain tissue of the plant can be used, e.g. in photosynthetically active tissue. Examples thereof are the ST-LS1 promoter (Stockhaus et al., 1987, Proc. Natl. Acad. Sci. USA 84, 7943-7947; Stockhaus et al., 1989, EMBO J. 8, 2445-2451), the Ca/b promoter (cf. e.g. US 5 656 496, US 5 639 952, Bansal et al., 1992, Proc. Natl. Acad. Sci. USA 89, 3654-3658) and the Rubisco SSU promoter (cf. e.g. US 5 034 322 and US 4 962 028). In addition, promoters which are active in the starch-storing organs of plants, such as the maize kernels in maize or the tubers in potatoes, can be used. For overexpressing the nucleic acid molecules of the invention in potato, the tuber-specific patatin gene promoter B33 (Rocha-Sosa et al., 1989, EMBO J. 8, 23-29) can, for example, be used. Seed-specific promoters have already been described for various plant species, e.g. the USP promoter of *Vicia faba,* which guarantees a seed-specific expression in *V. faba* and other plants (Fiedler et al., 1993, Plant Mol. Biol. 22, 669-679; Bäumlein et al., 1991, Mol. Gen. Genet. 225, 459-467).
Moreover, fruit-specific promoters as described in WO 91/01373 can also be used. Promoters for an endosperm-specific expression, such as the glutelin promoter (Leisy et al., 1990, Plant Mol. Biol. 14, 41-50; Zheng et al., 1993, Plant J. 4, 357-366), the HMG promoter from wheat, the USP promoter, the phaseolin promoter or promoters of zein genes from maize (Pedersen et al., 1982, Cell 29, 1015-1026; Quatroccio et al., 1990, Plant Mol. Biol. 15, 81-93) are particularly preferred. The shrunken-1-promoter (sh-1) from maize (Werr et al., 1985, EMBO J. 4, 1373-1380) is particularly preferred.
In addition, there may be a terminator sequence. This sequence is responsible for the correct termination of the transcription and the addition of a poly-A tail to the transcript, which has the function of stabilising the transcripts. Examples for such elements are the 3'-untranslated regions comprising the polyadenylation signal of the nopaline synthase gene (NOS gene) or the octopine synthase gene (see e.g. Gielen et al., 1898, EMBO J. 8, 23-29) from Agrobacteria or the 3'-untranslated regions of the genes of the storage proteins from soybean, as well as the genes of the small subunit of ribulose-1,5-bisphospate-carboxylase (ssRUBISCO).

In a preferred embodiment, a genetically modified plant cell according to the present invention which produces a starch with an increased amylose content in comparison to a corresponding non-genetically modified plant cell, and which is characterized in that it is genetically modified by the introduction of a nucleic acid molecule which encodes a PTST protein, and which is expressed in said plant cell, is further characterized in that it is also genetically modified with and expresses a nucleic acid molecule encoding a GBSS protein. Thus, such a plant cell is characterized in that it shows an increase both PTST and GBSS proteins in comparison to a corresponding non- genetically modified plant cell.
As described above, GBSS stands for granule-bound starch synthase. This protein is characterized in that it is located in the plastids, and is bound to the starch granule. It uses ADP-glucose to elongate amylose. GBSS proteins are known to exist in a multitude of plant species including, e.g., *Arabidopsis thaliana, Oryza sativa, Triticum aestivum, Triticum durum, Triticum spelta, Triticum turgidum, Tritcum turgidum* subsp. dicoccon, *Triticum urartu, Triticum moococcum, Aegilops crassa, Aegilops longissima, Aegilops searsii, Aegilops speltoides, Aegilops tauschii, Aegilops ventricos, Sorghum bicolor, Zea mays, Solanum tuberosum, Pisum sativum, Vigna radiate, Guillardia theta, Ipomoea batatas, Gracilaria tenuistipitata Chlamydomonas reinhardtii,* and nucleotide sequences encoding GBSS are available for most of these species, or can be obtained by applying routine techniques. In principle, any known GBSS can be employed in the context of the present invention for expression in genetically modified plant cells. In a preferred embodiment a GBSS protein is employed which is homologous to the plant cell to be transformed.

An increase in GBSS protein preferably means an increase in the activity of GBSS. GBSS activity can be measured by a person skilled in the art, e.g. though the incorporation of radiolabelled ADP-Glucose into isolated starch granules, as described in Vos-Scheperkeuter et al. (1986, Plant Physiol. 82, 411-416), Denyer et al. (1995, Plant Cell Environ. 18, 1019-1026) or Zeeman et al. (2002, Plant Physiol. 128, 1069-1076). Alternatively, an increase in GBSS protein means an increase in the amount of GBSS protein present in the genetically modified plant cell. The amount of GBSS protein can be determined by methods well known in the art, such as immunological assays, e.g. Western Blot or quantitative mass-spectrometry-based proteomic approaches. An increase in the amount of GBSS protein preferably means an increase of at least 10%, more preferably of at least 10%, more preferably of at least 20%, even more preferably of at least 50% and particularly preferred by of at least 100% when compared to a corresponding non-genetically modified plant cell.

In a further preferred embodiment, a genetically modified plant cell according to the invention is also characterized in showing an increased amount of transcripts encoding a GBSS protein in comparison to a corresponding non-genetically modified plant cell, i.e. a corresponding plant which is not genetically modified by the introduction of a nucleic acid molecule encoding a GBSS protein which is expressed in said plant cell. The term "an increased amount of transcripts encoding a GBSS protein" means that in the genetically modified plant cell according to the present invention, the amount of transcripts encoding a GBSS protein is increased in comparison to a corresponding non-genetically modified plant cell. Such an increase can be detected by methods known to the person skilled in the art, e.g. by Northern Blot or RT-PCR. The term "increased amount" preferably means that the amount of transcripts encoding the GBSS protein is increased by at least 10%, more preferably by at least 20%, even more preferably by at least 50% and particularly preferred by at least 100% when compared to a corresponding non-genetically modified plant cell. The genetically modified plant cell according to the present invention is characterized in that it is also transformed with a nucleic acid molecule encoding a GBSS protein that provides for expression in plant cells. The term "transformed" means that the nucleic acid encoding a GBSS protein is stably introduced into the genome of the genetically modified plant cell and is controlled by a promoter active in plant cells, in particular in plant cells in which it is desired that the GBSS protein is to be expressed. The genetically modified plant cells according to the present invention differ from naturally occurring plant cells, in particular from the corresponding non-genetically modified cells, in that at least one copy of the nucleic acid encoding a GBSS protein is integrated into their genome of the genetically modified plant, possibly in addition to naturally occurring copies. Preferably this/these additional copy/copies is/are integrated at a location in the genome of the plant cell at which the possibly existing endogenous gene encoding a GBSS protein does not naturally occur. This may be determined, e.g., by Southern Blot analysis.

In a preferred embodiment, the nucleic acid molecule encoding a GBSS protein is introduced into the plant cells, stably integrated in its genome and is foreign with respect to the plant cell. The term "foreign" means that such a nucleic acid molecule does not naturally occur in the plant cell from which the genetically modified cell according to the invention is derived by the introduction of the nucleic acid molecule. If the GBSS protein that is encoded by the nucleic acid molecule is a GBSS which is endogenously found in the plant cell (i.e. a GBSS that does naturally occur in the plant cell), then the term "foreign" means that the nucleic acid molecule contains at least one component that does not naturally occur in said plant cell. Preferably, said component is a regulatory element for expression in plant cells, preferably a promoter, which is naturally not part of the GBSS gene of the plant cell into which the nucleic acid molecule is introduced. In other words, it is a regulatory element for expression in plant cells that is heterologous with respect to the nucleotide sequence encoding the GBSS protein to be introduced into the plant cell.
A GBSS protein is characterized in that it has a chloroplast transit peptide which ensures localization of the protein in the plastids. In one embodiment, the nucleic acid molecule that is introduced into the plant cells encodes a GBSS protein that comprises its natural chloroplast transit peptide. In another embodiment the nucleic acid which is introduced into the plant cells encodes a GBSS protein in which the naturally occurring chloroplast transit peptide is replaced by a peptide from another protein which ensures the translocation of the protein into the plastids, in particular the chloroplasts, e.g. a GBSS protein from another species or from a different protein.

The present invention also relates to genetically modified plants containing genetically modified plant cells according to the present invention that produce a starch with an increased amylose content in comparison to a corresponding non-genetically modified plant cell and which are characterized in that they are genetically modified by the introduction of a nucleic acid molecule encoding a PTST protein, which is expressed in said plant cells.
In principle, the genetically modified plant may be of any possible plant species. It may be a monocotyledonous or of a dicotyledonous plant. Preferably the plant is a useful plant, such as a vegetable plant (e.g. tomatoes) or a starch storing plant such as those mentioned above. It is also conceivable that the plant is, e.g. an oil-producing plant, such as oils seed rape or sunflower, or hemp, flax or pastures like white clover, ryegrass or alfalfa.

The genetically modified plant cells and genetically modified plants according to the invention can be prepared according to methods well-known to the person skilled in the art. Such methods include but are not limited to Agrobacterium-mediated transformation, electroporation, biolistic methods, chemically induced DNA absorption into protoplasts the macroinjection of DNA in the inflorescence, the microinjection of DNA in microspores and pro-embryos, the DNA absorption through germinating pollens and the DNA absorption in embryos by swelling.

Genetically modified plants according to the present invention can be obtained by regenerating transformed plant cells by methods well-known to the person skilled in the art. The plants obtainable by means of regeneration of the genetically modified plant cells of the invention and containing such plants are also a subject matter of the present invention.

The present invention also relates to parts of the genetically modified plants according to the present invention which produce a starch with an increased amylose content, said parts containing genetically modified plant cells according to the invention as described herein-above. Such parts include, e.g., harvestable parts, preferably starch storing organs such as tubers, grains, stems, leaves or roots.
The present invention also relates to propagation material of a genetically modified plant according to the invention containing genetically modified cells according to the invention. Such propagation material includes, e.g. seeds, root stocks, tubers, cuttings and fruits.

The present invention also relates to a method for the production of a genetically modified plant which synthesizes a starch with an increased amylose content containing the step of genetically modifying plant cells by introducing into the plant cells a nucleic acid molecule which encodes a PTST protein and which expresses said PTST protein in plant cells, and regenerating from said plant cells into which said nucleic acid molecule is introduced whole plants, wherein the regenerated plant is characterized in that it contains genetically modified plant cells containing and expressing said nucleic acid molecule and synthesizing a starch with an increased amylose content in comparison with corresponding non-genetically modified plant cells.
As regards the plant cells, the plants, the amylose content and the nucleic acid molecule, the same applies in the context of the method according to the invention as has been set forth herein-above in connection with the genetically modified plant cells according to the present invention synthesizing a starch with an increased amylose content.

The present invention also relates to a method for producing a starch with an increased amylose content comprising the step of extracting starch from a plant according to the present invention containing plant cells according to the present invention synthesizing a starch with an increased amylose content or from parts of such a plant.
Such a method may also further comprise the step of harvesting a plant according to the present invention containing plant cells according to the present invention synthesizing a starch with an increased amylose content or harvesting parts of such a plant. It may also further comprise the step of cultivating a plant according to the present invention containing plant cells according to the present invention synthesizing a starch with an increased amylose content.

The present invention also relates to a method for producing a starch with an increased amylose content comprising the step of:
extracting from plants according to the invention as described herein-above which produce a starch with an increased amylose content or from starch storing parts of said plants the starch. Preferably, such a method also comprises the step of harvesting the cultivated plants and/or the starch-storing parts of said plants before the starch is extracted and, more preferably, also the step of cultivating plants of the invention prior to harvesting them.
Thus, the present invention also relates to a method for producing a starch with an increased amylose content comprising the steps of
(a) harvesting genetically modified plants as described herein-above containing plant cells which are genetically modified by the introduction of a nucleic acid molecule which encodes a PTST protein, and which is expressed in said plant cells, wherein said plant cells synthesize a starch with an increased amylose content in comparison to corresponding non-genetically modified plant cells; or starch storing parts of said plants; and
(b) extracting from the plants or from the starch storing parts of said plants the starch.
Moreover, the present invention also relates to a method for producing a starch with an increased amylose content comprising the steps of
(a) culturing a genetically modified plant as described herein-above containing plant cells which are genetically modified by the introduction of a nucleic acid molecule which encodes a PTST protein and which is expressed in said plant cells, wherein said plant cells synthesize a starch with an increased amylose content in comparison to corresponding non-genetically modified plant cells; and
(b) harvesting the cultivated plants of (a) or starch storing parts of said plants; and
(c) extracting from the plants or from the starch storing parts of said plants the starch.

Methods for extracting starch from plants or from starch storing parts of plants are well known to the person skilled in the art and have been described herein-above.

The present invention also relates to the starch produced by a method according to the invention as described herein-above.

The present invention also relates to the use of a nucleic acid molecule encoding a PTST protein for the production of a genetically modified plant cell which is genetically modified so as to overexpress said PTST protein and which synthesizes a starch having an increased amylose content when compared to a corresponding non-genetically modified plant.

The present invention also relates to a method for increasing the amylose content of starch produced in plant cells, comprising the step of increasing in said plant cells the expression of a PTST protein.
- **Figure 1**: shows the structure of a starch granule and schematically the components of plant starch
- **Figure 2**: shows the enzymes responsible for the synthesis of the different components of starch
- **Figure 3**: shows the overall structure of the PTST protein with the N-terminal chloroplast transit peptide, the coiled-coil domain and the Carbohydrate-Binding Module (CBM).
- **Figure 4**: shows schematically an assay for testing the binding of a PTST protein to starch.
- **Figure 5**: shows the set-up of a pulldown assay using recombinantly produced PTST and GBSS proteins.
- **Figure 6**: shows the results of the determination of the starch content and amylose content of PTST T-DNA insertion mutants (*ptst*-3 and *ptst*-4) of Col and Ws wild-type plants and of a GBSS T-DNA insertion mutant (gbss).
- **Figure 7**: shows the SDS-PAGE analysis of proteins isolated from starch granules of the ptst-3 and gbss mutants and Col wild type plants using silver staining and immunoblotting for GBSS.
- **Figure 8**: shows the set-up and the results of a pull-down assay using recombinantly produced PTST and GBSS proteins.
- **Figure 9**: shows the set-up and the results of an assay for testing the binding of a PTST protein to starch.
- **Figure 10**: shows the results of expression of fluorescently-tagged and transiently expressed wild type GBSS and PTST proteins of *A. thaliana* in tobacco epidermal cells.
- **Figure 11**: shows the results of expression of fluorescently-tagged and transiently expressed wild type GBSS and PTST proteins and PTST W217/255A mutant protein of A. *thaliana* in tobacco epidermal cells.
- **Figure 12**: shows the results of expression of fluorescently-tagged and transiently expressed wild type GBSS1 and GBSS2 and PTST proteins of rice in tobacco epidermal cells.

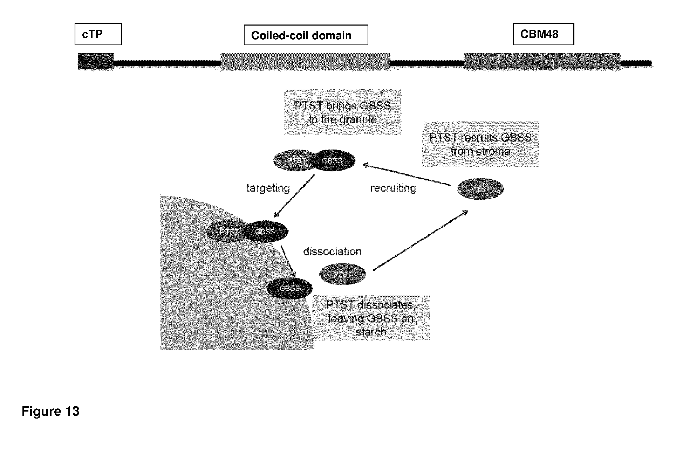

- **Figure 13**: shows a model for the interaction of PTST and GBSS. GBSS interacts with PTST, and PTST transports GBSS to the starch granule. PTST was always mainly detected in the chloroplast stroma. Hence, PTST must dissociate with the granule and GBSS and return to the stroma once the targeting process is complete. Once in the stroma, PTST can recruit another GBSS molecule.
- **Figure 14**: shows: (A) Detection of PTST and GBSS in soluble and insoluble protein fractions of plant leaves. (B) Detection of PTST and GBSS in soluble and insoluble protein fractions of leaves of *A. thaliana* wild type and *ptst*-3 mutant plants constitutively overexpressing GBSS.
- **Figure 15**: shows schematically the structure of the T-DNA insertion mutants *ptst*-1 to 4.
*ptst-3:*
- T-DNA insertion line from the Salk collection
- Ecotype background: Columbia
- Transformation vector: pROK
- Insertion in 3^{rd} exon
*ptst-4:*
- T-DNA insertion line from the Versailles (INRA) collection
- Ecotype background: Wassilewskija
- Transformation vector: pGKB5
- Insertion in 9^{th} (final) exon

In this specification, a number of documents including patent applications are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention will now be described by reference to the following examples which are merely illustrative and are not to be construed as a limitation of the scope of the present invention.

### EXAMPLES

### Plant materials and growth conditions

For all experiments, *Arabidopsis thaliana* plants were grown on soil in a controlled environment chamber (Percival AR-95L, CLF Plant Climatics, Wertingen, Germany; OR Kälte 3000, Landquart, Switzerland) under a 12-h light:12-h dark cycle. Light intensity was set to 150 µmol photons m⁻² s⁻¹, temperature was set to 22°C, and relative humidity was set to 65%.

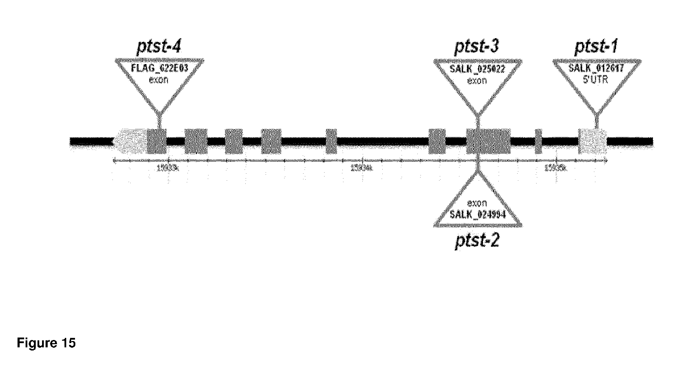

The following T-DNA insertion mutants were used in this study: *ptst-3* (SALK_025022; ecotype background Columbia (Col)), *ptst-4* (FLAG_622E03; ecotype background: Wassilewskija (WS)), and *gbss* (GABI_914G01; ecotype background Columbia (Col)). Figure 15 shows schematically the structure of these T-DNA insertion mutants.

### Starch content and iodine staining

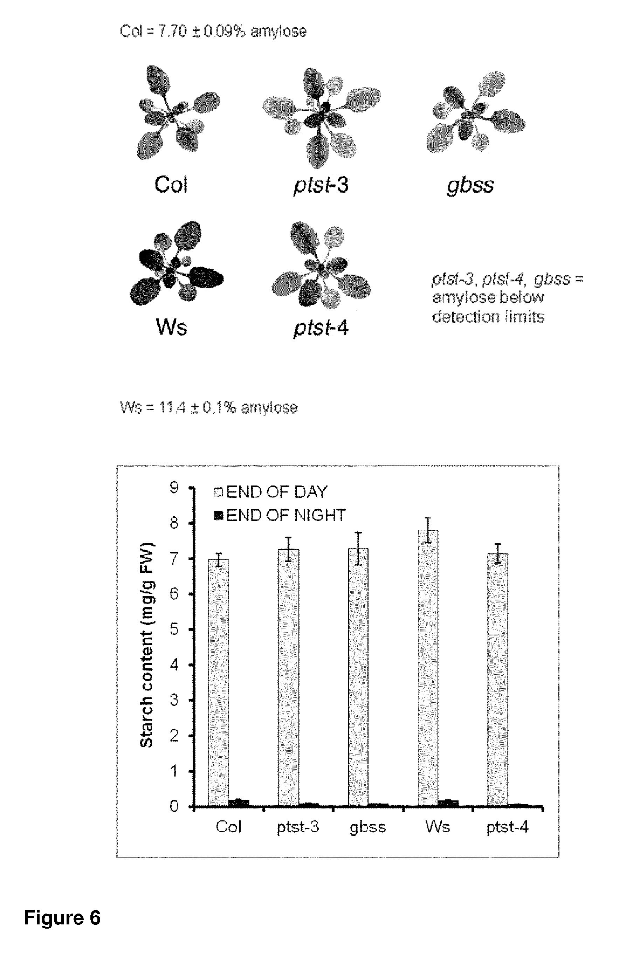

Starch was quantified according to the method of Smith & Zeeman (Quantification of starch in plant tissues. Nat. Protoc. 1 (2006), 1342-1345). Briefly, entire rosettes of 4-week-old plants were homogenised in 0.7 M perchloric acid. The insoluble material was pelleted by centrifugation, washed three times in 80% (*v*/*v*) ethanol, and resuspended in water. The starch in the insoluble fraction was gelatinised at 95°C for 15 min, and subsequently digested to glucose at 37°C using α-amylase and α-glucosidase (Roche, Basel, Switzerland). Starch content (in glucose equivalents) was then determined by quantifying glucose with a hexokinase/glucose-6-phosphate dehydrogenase-based assay (Roche).
For iodine staining of plant material, rosettes were harvested and decolorised in 80% (*v*/*v*) ethanol. Excess ethanol was removed by rinsing in water, before staining in Lugol solution (Sigma-Aldrich, Buchs, Switzerland). Plants were destained in water for several hours to enable the visualisation of the amylose-I₂ complex in starch. The results of these experiments are shown in Figure 6. From this Figure it is evident that the starch contents of the *ptst* mutants are identical to the starch content of wild type plants, while the starch composition (amylose content) is altered. Whilst both wild type plants stained a dark blue/black color, both ptst mutants stained a lighter brown colour. This staining was very similar to that of the gbss mutant, which is known to have no amylose. Starch granules were isolated from these plants and the amylose in these lines was quantified. The wild-type plants had 7.7% or 11.4% amylose for Col and Ws ecotypes respectively. No amylose was detected in either ptst line (see Figure 6). Taken together, these data show that the loss-of-function mutation of PTST results in altered starch composition (no amylose) without an alteration in starch content.

### Purification of starch granules from Arabidopsis

Entire rosettes of 4-week-old plants were homogenised in starch extraction medium (50 mM Tris-HCl, pH 8, 0.2 mM EDTA and 0.5% (*v*/*v*) Triton X-100) using a Waring blender. Insoluble material was resuspended in the extraction medium and sequentially filtered through 30 µm and 15 µm nylon nets. Starch granules were separated from the filtrate at 2,500g for 15 min over a 95% (*v*/*v*) Percoll cushion (Sigma-Aldrich). The starch pellet was then washed in 0.5% (*w*/*v*) sodium dodecyl sulfate (SDS). Excess SDS was removed by extensively washing the pellet in water. The pellet was then washed once in 80% (*v*/*v*) ethanol, and dried under vacuum overnight.

### Determination of apparent amylose content

Amylose content of the purified starch granules was determined using the iodine colourimetry method, following the protocol described in Hostettler et al. 2011 (Methods Mol. Biol. 775, 387-410).

### Extraction of granule-bound proteins

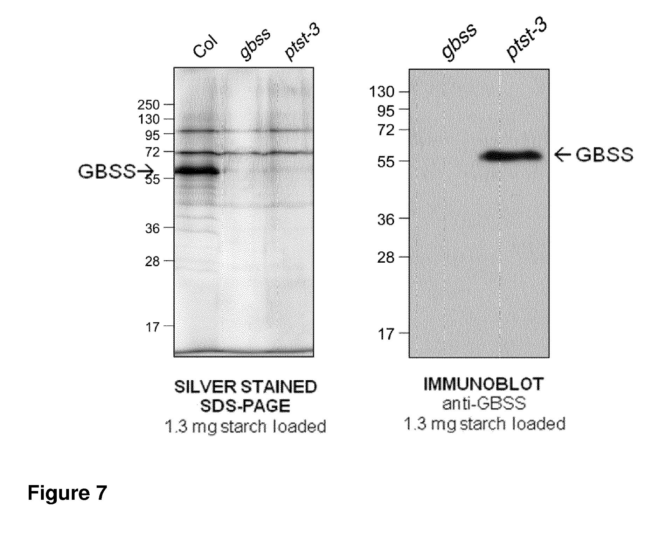

Granule-bound proteins were extracted from the purified starch granules as follows: Dried granules were resuspended at a concentration of 33 mg/mL in SDS-PAGE loading medium (50 mM Tris-HCl, pH 6.8, 2% (*w*/*v*) SDS, 100 mM DTT, 3% (*v*/*v*) glycerol, 0.005% (*w*/*v*) bromophenol blue), and heated at 95°C for 10 min. Gelatinised starch was then pelleted at 20,000*g* for 10 min. Proteins in the supernatant were separated on SDS-PAGE gels. For silver staining of protein gels, the gels were stained with the Silver Stain Plus kit (Bio-rad, Cressier, Switzerland). For immunoblotting, proteins were transferred onto a PVDF membrane, and probed with either an anti-GBSS (raised against the purified *At*GBSS recombinant protein; see below), or anti-PTST (Lohmeier-Vogel et al. loc. cit.) antibodies.
The results of these experiments for the PTST and GBSS mutants as well as for the corresponding wild type plants are shown in Figure 7. As expected, GBSS was the most abundant protein inside starch granules of wild type ('Col') plants. The GBSS band was missing in the *gbss* mutant, indicating it is a knock-out allele. Surprisingly, it was found that GBSS was also missing in *ptst* mutant starch granules. Immunoblotting was used as a more sensitive approach to detect GBSS. Immunoblotting revealed that there is some GBSS present in the starch granules of ptst mutants (see Figure 7). However, since this band can only be detected using immunoblotting and not with silver staining, it should be noted that the amount of GBSS is very low. This indicates that the loss of PTST results in a dramatic reduction of GBSS from the starch granule.

### Recombinant protein expression in E. coli

For GST-tagged PTST recombinant protein expression in *E*. *coli,* a PTST-cTP::pGEX-4T-2 IPTG-inducible expression vector was cloned as follows: A full length cDNA clone encoding the PTST protein was obtained from the RIKEN Bioresource Center (http://www.brc.riken.jp) (pda04554, RAFL08-09-K08). The region encoding the PTST protein (lacking the predicted chloroplast transit peptide: amino acids 1-44) was amplified with BamHI and NotI restriction sites using the following primers, 5'-ATGGATCCGCTTCTACTCGAAAACATTAC-3' (SEQ ID NO: 5) and 5'-TAGCGGCCGCCTATTCCACCACTAAAACATTG-3' (SEQ ID NO: 6), and cloned into the pGEX-4T-2 vector (GE Healthcare, Glattbrugg, Switzerland). Point mutations were generated using the QuikChange Site-Directed Mutagenesis Kit (Agilent Technologies, Basel, Switzerland) according to the manufacturer's instructions.

For GBSS recombinant protein expression in *E*. *coli,* a GBSS-cTP::pET24a+ IPTG-inducible expression vector was constructed. Arabidopsis cDNA was prepared from wild type Columbia plants using the RNeasy Plant total RNA extraction kit (Qiagen, Basel, Switzerland) and Wizard Reverse transcription kit (Promega, Wallisellen, Switzerland). The cDNA region encoding the GBSS protein (lacking the predicted chloroplast transit peptide; amino acids 1-79) was amplified from the cDNA preparation with NheI and XhoI restriction sites using the following primers: 5'-ATGCTAGCTGTGAGAAAGGAATGTCTGTG-3' (SEQ ID NO: 7) and 5'-ATCTCGAGCGGCGTCGCTACGTTCTC -3' (SEQ ID NO: 8). The amplicon was cloned into the pET24a+ vector (Novagen/Merck, Zug, Switzerland) in frame with the C-terminal His-tag.

*E. coli* BL21 (DE3) CodonPlus cells (Agilent Technologies) harbouring the expression vector plasmids were cultured in LB medium at 37°C. Protein expression was induced by adding 1 mM IPTG, and grown overnight at 20°C or 18.5°C for GST-PTST and GBSS respectively. For GST-PTST purification, cells were resuspended in lysis medium (50 mM Tris-HCl, pH 7.5, 300 mM NaCl, 2 mM DTT, 1 mg/mL lysozyme, and Complete Protease Inhibitor Cocktail (Roche)) and lysed with a microfluidiser (Microfluidics, Westwood, MA, USA). The lysate was spun at 20,000*g* for 10 min, and the supernatant was incubated with Glutathione Sepharose 4B resin (GE Healthcare). The resin was washed five times in triton wash medium (50 mM Tris-HCl, pH 7.5, 300 mM NaCl, 2 mM DTT, 0.5% (*v*/*v*) Triton X-100), five times in wash medium (50 mM Tris-HCl, pH 7.5, 300 mM NaCl, 2 mM DTT), and subsequently eluted in elution medium (50 mM Tris-HCl, pH 7.5, 300 mM NaCl, 10 mM reduced glutathione). Free GST was also purified as described for GST-PTST from cells harbouring the empty pGEX-4T-2 vector plasmid. For GBSS purification, the recombinant protein was purified from the lysate using Ni²⁺-NTA agarose (Qiagen) as described previously (Seung et al., 2013, J. Biol. Chem. 288, 33620-33633).

Fractions containing GST-PTST, free GST or GBSS were pooled and stored in 50 mM Tris-HCl, pH 7, 10% (*v*/*v*) glycerol and 2 mM DTT. Protein concentration was determined using the Bradford assay reagent (Bio-rad).

### Pull-down assay with recombinant proteins

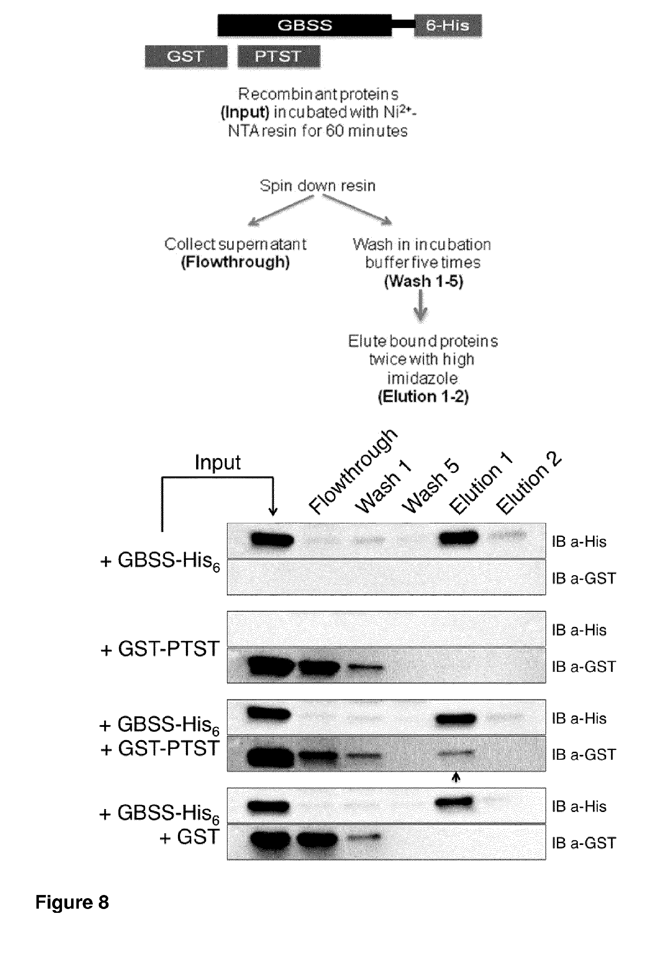

Purified recombinant proteins (1 µg each) were incubated together with Ni²⁺-NTA resin (Qiagen) for 60 min at room temperature in binding medium (50 mM Tris-HCl, pH 7.5, 300 mM NaCl, 10 mM imidazole, 0.1% (*w*/*v*) BSA). The resin was washed five times in wash medium (50 mM Tris-HCl, pH 7.5, 300 mM NaCl, 20 mM imidazole, 0.1% (*w*/*v*) BSA, 0.05% (*v*/*v*) NP-40), before eluting in elution medium (50 mM Tris-HCl, pH 7.5, 300 mM NaCl, 250 mM imidazole). Proteins were detected by SDS-PAGE followed by immunoblotting with anti-GST (Abcam, Cambridge, UK) or anti-PentaHis (Qiagen) antibodies.
The results of these experiments are shown in Figure 8. GBSS-His interacted strongly with the resin and was present at a high abundance in the elution fractions (detected by immunoblotting with an anti-His antibody). The GST-PTST protein has no His-tag, and therefore did not interact with the resin. Hence, it is absent in the elution fractions (detected by immunoblotting with an anti-GST antibody). However, when the GBSS-His and GST-PTST were incubated together with the Ni-NTA resin, both the GBSS-His and GST-PTST were present in the elution fractions. This shows that PTST must interact directly with GBSS. Since the GST-tag is a relatively large protein, the pulldown assay was also performed with GBSS-His and free GST as a control. No interaction between free GST and GBSS-His was detected.

### Glucan-binding assay

Recombinant GST-PTST (1 µg) was incubated in binding medium (50 mM HEPES-NaOH, pH 7.5, 2 mM MgCl2, 1 mM DTT, 0.1 % BSA (w/v), 0.01 % (v/v) Triton X-100) for 30 min at room temperature with maize starch (WT or waxy; Sigma-Aldrich, Buchs, Switzerland). Sephadex G-10 resin (GE Healthcare) was used as a non-glucan control. The reactions were mixed end-over-end on a spinning wheel during the incubation. The substrates were pelleted at 5,000g for 30 seconds, and unbound proteins were collected in the supernatant. The substrates were then washed three times in binding medium, and bound proteins were eluted in elution medium (50 mM HEPES-NaOH, pH 7.5, 2 mM MgCl2, 1 mM DTT, 2% (w/v) SDS). Proteins were detected by SDS-PAGE followed by immunoblotting with an anti-GST (Abcam) antibody.

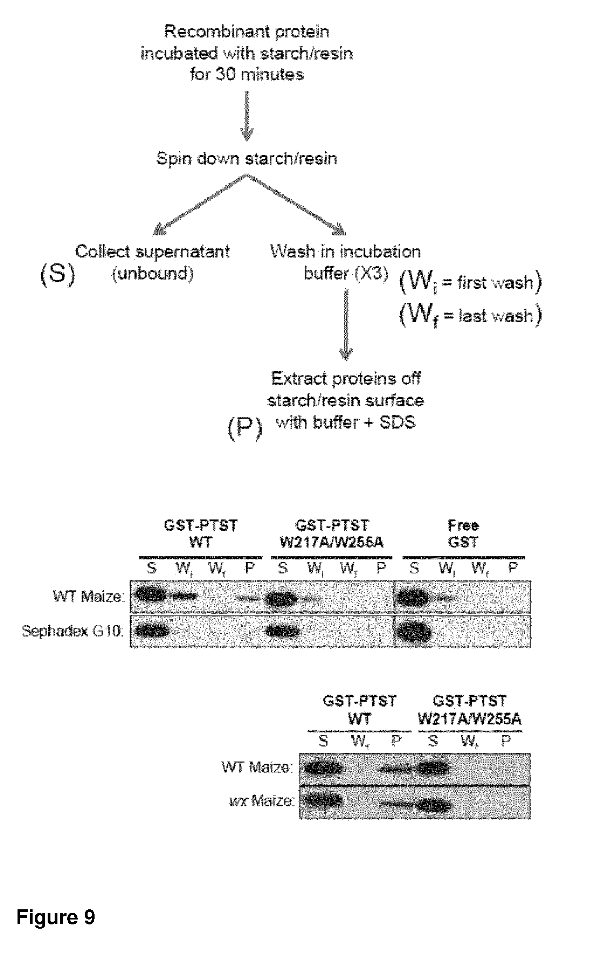

Two variants of the GST-PTST protein were tested: the wild type protein (WT), as well as a W217A/W255A site-directed mutant. The latter has two key tryptophan residues in the CBM48 domain mutated to alanine residues. Both tryptophan residues have been shown to be required for starch binding. The results of these experiments are shown in Figure 9. It was found that the WT PTST protein interacted with both WT and waxy maize starches. However, the interaction seemed relatively weak as only a fraction of the protein in the assay was found in the pellet with the starch granules. This interaction was abolished in the W217A/W255A mutant. Additionally, free GST protein did not interact with starch under these conditions, providing further evidence that the observed interaction is through PTST. No interaction was seen with the sephadex resin for any protein, showing that the observed interactions were glucan-specific.
From the findings that GBSS can interact directly with PTST and that PTST can interact with starch through the CBM48 domain it was hypothesised that PTST may act like a 'bridge' between the GBSS protein and the starch granule. To test this hypothesis, both the Arabidopsis PTST and GBSS proteins were expressed in tobacco epidermal cells with two different fluorescent protein tags (YFP and CFP respectively).

### Transient expression of fusion proteins in tobacco

The full coding sequence for Arabidopsis PTST was amplified from the cDNA clone described above with the following primers: 5'-ATGGGATGTGTACCCAGAATTG-3' (SEQ ID NO: 9) and 5'-TTCCACCACTAAAACATTGTTCTC-3' (SEQ ID NO: 10), and cloned into the Gateway-compatible entry vector, pCR8 using the pCR8/GW/TOPO TA kit (Invitrogen, Basel, Switzerland). The PTST and GBSS constructs were recombined into the Gateway destination vectors, pB7YWG2.0 (YFP; Karimi et al., 2002 Trends Plant Sci. 7, 193-195) and pEarleyGate102 (CFP; Earley et al., 2006, Plant J. 45, 616-29), respectively, in frame with the C-terminal fluorescent protein tags.
For experiments with rice GBSS and PTST isoforms, full coding sequences for rice GBSS1 (Os06g04200), GBSS2 (Os07g22930) and PTST (Os02g04330) were synthesized using a gene synthesis service provided by Biomatik (Ontario, Canada). The following primers, including *attB1* and *attB2* recombination sites (underlined), were used to amplify the coding sequences:
For OsGBSS1 - 
5’-
GGGGACAAGTTTGTACAAAAAAGCAGGCTTCACCATGTCGGCTCTCACCACGT
C-3’ (SEQ ID NO: 11)
 and 
5'-GGGGACCACTTTGTACAAGAAAGCTGGGTCAGGAGCAGCCACGTTCTCC-3'
(SEQ ID NO: 12);
for OsGBSS2 - 
5’-
GGGGACAAGTTTGTACAAAAAAGCAGGCTTCACCATGGCTAGAACAATGGGTT
CAAC-3’ (SEQ ID NO: 13)
 and 
5'-GGGGACCACTTTGTACAAGAAAGCTGGGTCGGGAGTGGCCACGTTTTCC-3'
(SEQ ID NO: 14),
for OsPTST: 
5’-
GGGGACAAGTTTGTACAAAAAAGCAGGCTTCACCATGGAGTGTCTGACCACAA
GTTTTAC-3’ (SEQ ID NO: 15)
 and

5’-
GGGGACCACTTTGTACAAGAAAGCTGGGTCATTTACAACAAGTATATTGTTCTG
CATTGAAC-3’ (SEQ ID NO:16).

The amplicons were cloned into the Gateway-compatible entry vector, pDONR221 using the Gateway BP Clonase Enzyme Mix kit (Invitrogen, Basel, Switzerland). As above, the resulting OsGBSS1 and OsGBSS2 constructs were recombined into pEarleyGate102 in frame with the C-terminal CFP tag. The OsPTST construct was recombined into pB7YWG2.0 in frame with the C-terminal YFP tag.

Expression constructs were transformed into *Agrobacterium tumefaciens* (strain GV3101), and cultures were grown at 28 °C in LB medium supplemented with the appropriate antibiotics for 22-24 h. Cells were pelleted at 5000*g* for 15 min and resuspended in water to an OD₆₀₀ of 1. The cell suspension was then infiltrated into the intercellular spaces between abaxial epidermal cells of intact *Nicotiana benthamiana* leaves using a 1 mL plastic syringe. Infiltration of 0.2 mL into each spot yielded approx. 5 cm² coverage of leaf area. After infiltration, plants were placed away from strong light for 48 hours.

The plants were placed under illumination for 12 hours prior to microscopy. Images were acquired on a Zeiss LSM 780 confocal microscope (Carl Zeiss, Jena, Germany), with a 40X water-immersion lens with a 1.1 numerical aperture. YFP signals were monitored using an argon laser at 514 nm wavelength for excitation, and emitted light was captured between a wavelength window of 518 to 557 nm. CFP signals were monitored using an argon laser at 458 nm for excitation, and emitted light was captured between 462 to 500 nm. Autofluorescence of chlorophyll was monitored using an argon laser at 514 nm for excitation, and emitted light was captured between 662 to 721 nm. Images were processed on ImageJ software

### (http://rsbweb.nih.gov/ij/).

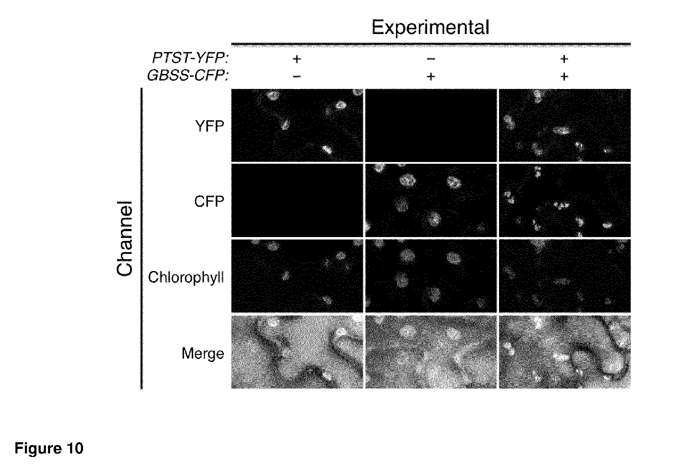

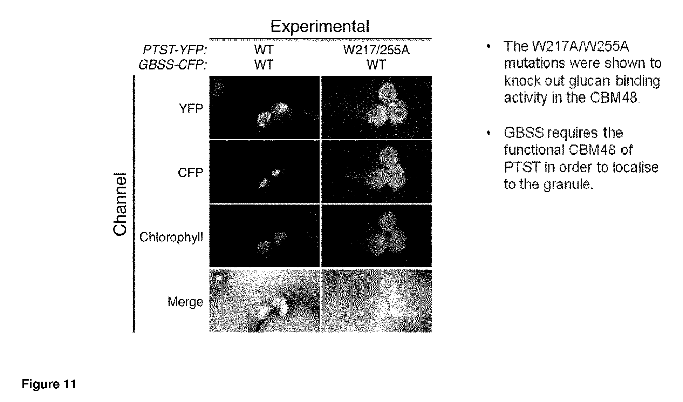

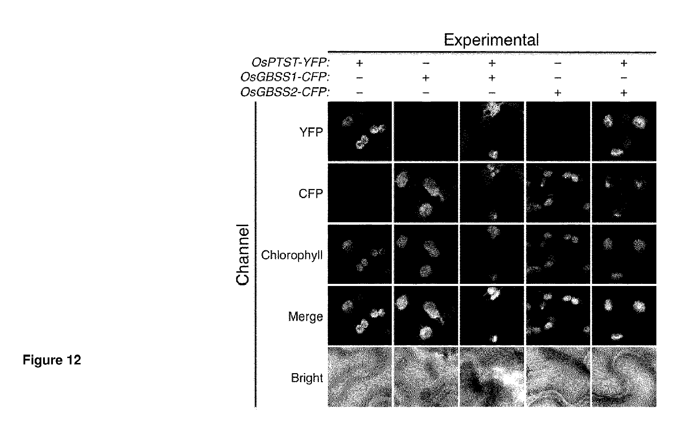

The results of these experiments are shown in Figure 10. When PTST-YFP was expressed alone, the YFP signal was found primarily in the chloroplast stroma (seen as the co-localization of the YFP signal with the chlorophyll autofluorescence signal). A similar localization was seen for GBSS-CFP, when GBSS-CFP was expressed alone. However, when both proteins were expressed together, the GBSS-CFP localized exclusively onto starch granules within the chloroplasts. The PTST-YFP signal remained in the stroma. These data suggest that PTST is required for GBSS localization onto starch granules.
The experiment was repeated with the W217A/W255A variant of the PTST protein. This variant carries mutations in the CBM48 domain and was shown to be unable to bind starch. The corresponding results are shown in Figure 11. Once again, when GBSS-CFP was expressed with the wild type PTST-YFP, all the CFP signal localized to starch granules within chloroplasts. However, when GBSS-CFP was co-expressed with the mutated PTST protein, the GBSS could not localize to the starch. These data show that a functional CBM48 is absolutely required for PTST to direct GBSS to the starch granule.
In rice, there are two GBSS isoforms: OsGBSS1 which is expressed specifically in the endosperm, and OsGBSS2, which is similar to Arabidopsis *thaliana* GBSS and shows stronger expression in leaf tissue. However, there is only one PTST gene in rice, which is expressed in both leaves and the endosperm. To address whether PTST transports both GBSS isoforms in rice to starch granules, we repeated the experiment above (Figure 11) with the rice proteins. The results of these experiments are shown in Figure 12. Not unlike the Arabidopsis isoform, the rice PTST localised to the chloroplast stroma. When expressed in the absence of OsPTST, both rice GBSS1 and GBSS2 showed a diffuse stromal localisation. However, when co-expressed with OsPTST, both isoforms of GBSS localised exclusively to the starch granule. This suggests that PTST is capable of transporting both isoforms of GBSS to the granule.

### Generation of transgenic GBSS overexpression lines

The full-length coding sequence of the Arabidopsis GBSS protein was amplified from an Arabidopsis cDNA preparation (described above), with the following primers: 5'-ATGGCAACTGTGACTGCTTCTTCTAA-3' (SEQ ID NO: 17) and 5'-CGGCGTCGCTACGTTCTC-3' (SEQ ID NO: 18). The amplicon was cloned into the Gateway-compatible entry vector, pCR8 using the pCR8/GW/TOPO TA kit (Invitrogen). The construct was recombined into the Gateway-compatible pEarleyGate103 vector (Earley et al. (2006), loc. cit.) in frame with the C-terminal GFP/His tag. This vector was introduced into Agrobacterium cells (strain: GV3101), and then transformed into wild-type Columbia and *ptst-3* plants as described previously (Zhang et al., 2006 Nat. Protoc. 1, 641-646). Screening was based on resistance to the herbicide, Basta. The analyses presented in this work were carried out on resistant T2 seedlings.

### Fractionation of soluble and insoluble proteins.

A 7 mm leaf disc was harvested from young leaves at the end of day and snap frozen in liquid N₂. The disc was homogenised using a pestle in soluble protein extraction medium (40 mM Tris-HCl, pH 6.8, 5 mM MgCl₂, Complete protease inhibitor (Roche)). Insoluble material was pelleted at 20,000*g*, and soluble proteins were collected in the supernatant. The pellet was washed once in soluble protein extraction medium, and subsequently resuspended in an equivalent volume of SDS-PAGE loading medium (see above). The suspension was then heated at 95°C for 5 min, and insoluble material was removed by centrifugation. GBSS-GFP was detected by SDS-PAGE followed by immunoblotting with an anti-GFP antibody (Clontech JL-8, Basel, Switzerland).

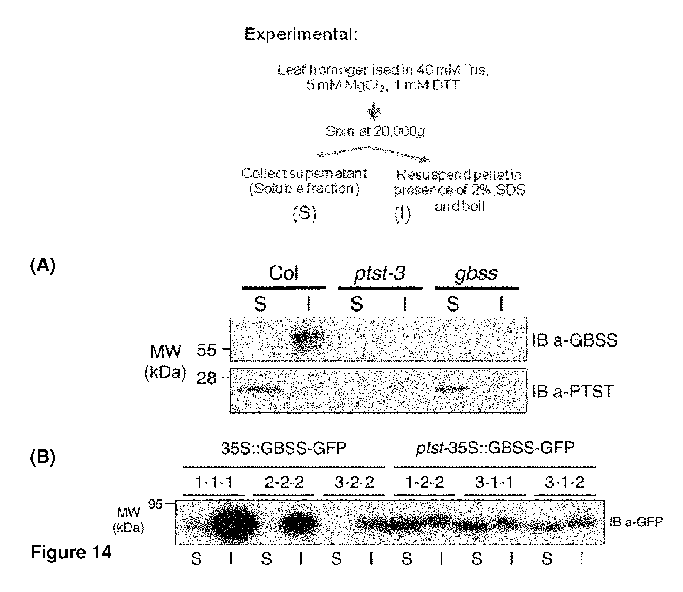

Figure 14 shows the results of the experiments for detecting GBSS and PTST proteins in the soluble and insoluble protein fractions obtained from leaves of plants. In order to verify whether the above proposed model is correct, experiments were carried out so as to detect 'free' GBSS in the ptst mutant. The protein was fractionated as described above - all starch-bound proteins would be detected in the insoluble fraction. PTST was exclusively detected in the soluble fraction, which supports the hypothesis that PTST dissociates from the granule once it has played its targeting role. In contrast, GBSS was detected exclusively in the insoluble fraction in the wild type plant (Col). This is consistent with its known localization inside the starch granule. However, in the ptst mutant, neither the soluble nor insoluble fractions had any detectable GBSS. This is not so surprising - it has long been speculated that 'free' GBSS is unstable as the protein has only been detected in its granule-bound form. To circumvent this problem, GBSS (with GFP tag) was constitutively overexpressed in Arabidopsis plants. When expressed in wild type (35S::GBSS-GFP lines), it was found that GBSS-GFP mainly localized in starch granules (insoluble fraction). Even in lines where expression was extremely strong (line #1-1-1), hardly any GBSS was found in the soluble fraction. In contrast, when the GBSS-GFP was overexpressed in mutant Arabidopsis plants lacking PTST (ptst-35S::GBSS-GFP lines), it was found that even under moderate levels of expression, GBSS-GFP was found mainly in the soluble fraction. This provides further evidence that PTST plays an important role in targeting GBSS to the starch granule.

## Claims

1. A method for preparing a plant cell which synthesizes a starch with a reduced amylose content said method comprising the step of genetically modifying a plant cell so that it shows a reduced expression of a PTST protein in comparison to a non-genetically modified plant cell.

2. The method of claim 1 wherein the PTST protein is **characterized by** the following features:
(a) It shows an amino acid sequence as shown in SEQ ID NO:2 or a sequence which is at least 32% identical to the sequence shown in SEQ ID NO:2;
(b) it is targeted to the chloroplasts;
(c) it has an N-terminal domain featuring a coiled-coil;
(d) it has a Carbohydrate-Binding Module (CBM) for interacting with carbohydrates located at the C-terminus;
(e) it can bind to starch;
(f) it can interact with GBSS (Granule Bound Starch Synthase).

3. The method of claim 1 or 2, wherein the genetic modification of the plant cell consists of a genetic modification selected from the group consisting of:
(a) deletion or disruption of an endogenous gene encoding a PTST protein;
(b) mutation of an endogenous PTST gene so as to encode a non-functional PTST protein;
(c) expression of an siRNA, an RNAi or a cosuppression RNA directed against transcripts encoding a PTST protein;
(d) expression of an antisense RNA which is complementary to transcripts encoding a PTST protein; and
(e) expression of a ribozyme directed against transcripts encoding a PTST protein.

4. Use of a nucleic acid molecule encoding a PTST protein or of a part of such a nucleic acid molecule for the preparation of a genetically modified plant cell which shows a reduced expression of said PTST protein in comparison to a non-genetically modified plant cell and which synthesizes a starch with a reduced amylose content.

5. A plant cell which is genetically modified so as to synthesize a starch with a reduced amylose content due to the reduced expression of a PTST protein in comparison to a non-genetically modified plant cell, wherein the genetic modification of the plant cell consists of a genetic modification selected from the group consisting of:
(a) deletion or disruption of an endogenous gene encoding a PTST protein;
(b) mutation of an endogenous PTST gene so as to encode a non-functional PTST protein;
(c) expression of an siRNA, an RNAi or a cosuppression RNA directed against transcripts encoding a PTST protein;
(d) expression of an antisense RNA which is complementary to transcripts encoding a PTST protein; and
(e) expression of a ribozyme directed against transcripts encoding a PTST protein.

6. A genetically modified plant comprising genetically modified plant cells of claim 5.

7. A method for producing a starch with a reduced amylose content comprising the step of extracting starch from a plant of claim 6 or of parts of a plant of claim 6.

8. The method of claim 7 which also comprises the step harvesting a plant of claim 6 or of parts of a plant of claim 6.

9. The method of claim 8 which also comprises the step of cultivating a genetically modified plant of claim 6.

10. A starch produced by a method according to any one of claims 7 to 9.

11. A genetically modified plant cell which produces a starch with an increased amylose content in comparison to a corresponding non-genetically modified plant cell and which is **characterized in that** it is genetically modified by the introduction of a nucleic acid molecule encoding a PTST protein which is expressed in said plant cell.

12. A genetically modified plant containing plant cells of claim 11.

13. A method for the production of a genetically modified plant which synthesizes a starch with an increased amylose content containing the step of genetically modifying plant cells by introducing into the plant cells a nucleic acid molecule which encodes a PTST protein and which expresses said PTST protein in plant cells and regenerating from said plant cells into which said nucleic acid molecule is introduced whole plants, wherein the regenerated plant is **characterized in that** it contains genetically modified plant cells containing and expressing said nucleic acid molecule and synthesizing a starch with an increased amylose content in comparison with corresponding non-genetically modified plant cells.

14. A genetically modified plant obtained by the method of claim 13.

15. A method for producing a starch with an increased amylose content comprising the step of extracting starch from a plant of claim 12 or 14 or of parts of a plant of claim 12 or 14.

16. The method of claim 15 which also comprises the step harvesting a plant of claim 12 or 14 or of parts of a plant of claim 12 or 14.

17. The method of claim 16 which also comprises the step of cultivating a genetically modified plant of claim 12 or 14.

18. A starch produced by a method according to any one of claims 15 to 17.

19. The genetically modified plant of claim 6 or 12 or 14 which is a starch storing plant.

20. The genetically modified plant of claim 6 or 12 or 14 which is rye, barley, wheat, oat, millet, rice, maize, peas, cow peas, wrinkled peas, chickpea, lentils, beans, mung bean, cassava, arrowroot, sweet potato, yarn, taro, potato, tomato, banana, sago or a starch storing alga.

21. Use of a nucleic acid molecule encoding a PTST protein for the production of a plant cell which is genetically modified so as to overexpress said PTST protein and which synthesizes a starch having an increased amylose content when compared to a corresponding non-genetically modified plant.


SEQUENCE LISTING

<110> ETH Zürich/ETH transfer

<120> Means and methods for modyfying the amylose content in plant starch

<160> 18

<170> BiSSAP 1.2

<210> 1
<211> 822
<212> DNA
<213> Arabidopsis thaliana

<220>
<221> source
<222> 1..822
<223> /mol_type="unassigned DNA"
      /organism="Arabidopsis thaliana"

<220>
<221> CDS
<222> 1..822
<223> /transl_table=1

<400> 1
atg gga tgt gta ccc aga att gaa ttt ggt tgt tca agc caa agc ctt      48
Met Gly Cys Val Pro Arg Ile Glu Phe Gly Cys Ser Ser Gln Ser Leu
1               5                   10                  15

act ctt tca tgg aac tta aga gca tgg aat ttg tgt aga ttg aac aca      96
Thr Leu Ser Trp Asn Leu Arg Ala Trp Asn Leu Cys Arg Leu Asn Thr
            20                  25                  30

atc tcc cat ttc cag aaa ctt cct tat cct ttg gta gct tct act cga     144
Ile Ser His Phe Gln Lys Leu Pro Tyr Pro Leu Val Ala Ser Thr Arg
        35                  40                  45

aaa cat tac aag aac agt ttg ctt ttg aag aga ttt tta gtt ggt gtt     192
Lys His Tyr Lys Asn Ser Leu Leu Leu Lys Arg Phe Leu Val Gly Val
    50                  55                  60

ggc act gag gaa tca tca ctg tca gaa gat tta cta gat gaa tct ctt     240
Gly Thr Glu Glu Ser Ser Leu Ser Glu Asp Leu Leu Asp Glu Ser Leu
65                  70                  75                  80

tct cga cct ctc act agt gat gag ttg aag tca ctg ctc att gat aca     288
Ser Arg Pro Leu Thr Ser Asp Glu Leu Lys Ser Leu Leu Ile Asp Thr
                85                  90                  95

gag aga tca aag ctt gtt aaa aaa ttg agt gaa gct aat caa cag aat     336
Glu Arg Ser Lys Leu Val Lys Lys Leu Ser Glu Ala Asn Gln Gln Asn
            100                 105                 110

cgg ttc ctc aag cgt cag gaa cat gag ata act aac atc aaa act gag     384
Arg Phe Leu Lys Arg Gln Glu His Glu Ile Thr Asn Ile Lys Thr Glu
        115                 120                 125

ctt gca ctt atg gag ctc gaa gtt cag gct ctg gtg aaa ctg gcg gaa     432
Leu Ala Leu Met Glu Leu Glu Val Gln Ala Leu Val Lys Leu Ala Glu
    130                 135                 140

gaa ata gca aac ctt ggt atc cca caa ggt tct aga aaa atc agt gga     480
Glu Ile Ala Asn Leu Gly Ile Pro Gln Gly Ser Arg Lys Ile Ser Gly
145                 150                 155                 160

aag tac att caa tcg cac ctt ctc tct cgt tta gac gct gtt caa aaa     528
Lys Tyr Ile Gln Ser His Leu Leu Ser Arg Leu Asp Ala Val Gln Lys

SEQLTXT.txt
165                 170                 175

aag atg aaa gaa caa ata aaa ggt gtc gag gct gca cag tca aag gag     576
Lys Met Lys Glu Gln Ile Lys Gly Val Glu Ala Ala Gln Ser Lys Glu
            180                 185                 190

gtt cat gtg ttc tgg att ggc atg gca gag agt gta caa gta atg gga     624
Val His Val Phe Trp Ile Gly Met Ala Glu Ser Val Gln Val Met Gly
        195                 200                 205

tcg ttc gat gga tgg agc caa cgt gag gat cta tcg cct gag tac tca     672
Ser Phe Asp Gly Trp Ser Gln Arg Glu Asp Leu Ser Pro Glu Tyr Ser
    210                 215                 220

gct tta ttc acc aaa ttc tcc aca acg tta ttc ctt cgt ccc ggg cgg     720
Ala Leu Phe Thr Lys Phe Ser Thr Thr Leu Phe Leu Arg Pro Gly Arg
225                 230                 235                 240

tac gag atg aag ttc tta gtg gat gga gaa tgg cag atc tca ccc gag     768
Tyr Glu Met Lys Phe Leu Val Asp Gly Glu Trp Gln Ile Ser Pro Glu
                245                 250                 255

ttt cct act tca gga gaa gga ttg atg gag aac aat gtt tta gtg gtg     816
Phe Pro Thr Ser Gly Glu Gly Leu Met Glu Asn Asn Val Leu Val Val
            260                 265                 270

gaa tag                                                             822
Glu


<210> 2
<211> 273
<212> PRT
<213> Arabidopsis thaliana

<220>
<223> [CDS]:1..822 from SEQ ID NO 1

<400> 2
Met Gly Cys Val Pro Arg Ile Glu Phe Gly Cys Ser Ser Gln Ser Leu
1               5                   10                  15
Thr Leu Ser Trp Asn Leu Arg Ala Trp Asn Leu Cys Arg Leu Asn Thr
            20                  25                  30
Ile Ser His Phe Gln Lys Leu Pro Tyr Pro Leu Val Ala Ser Thr Arg
        35                  40                  45
Lys His Tyr Lys Asn Ser Leu Leu Leu Lys Arg Phe Leu Val Gly Val
    50                  55                  60
Gly Thr Glu Glu Ser Ser Leu Ser Glu Asp Leu Leu Asp Glu Ser Leu
65                  70                  75                  80
Ser Arg Pro Leu Thr Ser Asp Glu Leu Lys Ser Leu Leu Ile Asp Thr
                85                  90                  95
Glu Arg Ser Lys Leu Val Lys Lys Leu Ser Glu Ala Asn Gln Gln Asn
            100                 105                 110
Arg Phe Leu Lys Arg Gln Glu His Glu Ile Thr Asn Ile Lys Thr Glu
        115                 120                 125
Leu Ala Leu Met Glu Leu Glu Val Gln Ala Leu Val Lys Leu Ala Glu
    130                 135                 140
Glu Ile Ala Asn Leu Gly Ile Pro Gln Gly Ser Arg Lys Ile Ser Gly
145                 150                 155                 160
Lys Tyr Ile Gln Ser His Leu Leu Ser Arg Leu Asp Ala Val Gln Lys
                165                 170                 175
Lys Met Lys Glu Gln Ile Lys Gly Val Glu Ala Ala Gln Ser Lys Glu
            180                 185                 190
Val His Val Phe Trp Ile Gly Met Ala Glu Ser Val Gln Val Met Gly
        195                 200                 205
Ser Phe Asp Gly Trp Ser Gln Arg Glu Asp Leu Ser Pro Glu Tyr Ser
    210                 215                 220
Ala Leu Phe Thr Lys Phe Ser Thr Thr Leu Phe Leu Arg Pro Gly Arg
225                 230                 235                 240
Page 2

SEQLTXT.txt
Tyr Glu Met Lys Phe Leu Val Asp Gly Glu Trp Gln Ile Ser Pro Glu
                245                 250                 255
Phe Pro Thr Ser Gly Glu Gly Leu Met Glu Asn Asn Val Leu Val Val
            260                 265                 270
Glu

<210> 3
<211> 85
<212> PRT
<213> Arabidopsis thaliana

<400> 3
Leu Val Lys Lys Leu Ser Glu Ala Asn Gln Gln Asn Arg Phe Leu Lys
1               5                   10                  15
Arg Gln Glu His Glu Ile Thr Asn Ile Lys Thr Glu Leu Ala Leu Met
            20                  25                  30
Glu Leu Glu Val Gln Ala Leu Val Lys Leu Ala Glu Glu Ile Ala Asn
        35                  40                  45
Leu Gly Ile Pro Gln Gly Ser Arg Lys Ile Ser Gly Lys Tyr Ile Gln
    50                  55                  60
Ser His Leu Leu Ser Arg Leu Asp Ala Val Gln Lys Lys Met Lys Glu
65                  70                  75                  80
Gln Ile Lys Gly Val
                85

<210> 4
<211> 82
<212> PRT
<213> Arabidopsis thaliana

<400> 4
Lys Glu Val His Val Phe Trp Ile Gly Met Ala Glu Ser Val Gln Val
1               5                   10                  15
Met Gly Ser Phe Asp Gly Trp Ser Gln Arg Glu Asp Leu Ser Pro Glu
            20                  25                  30
Tyr Ser Ala Leu Phe Thr Lys Phe Ser Thr Thr Leu Phe Leu Arg Pro
        35                  40                  45
Gly Arg Tyr Glu Met Lys Phe Leu Val Asp Gly Glu Trp Gln Ile Ser
    50                  55                  60
Pro Glu Phe Pro Thr Ser Gly Glu Gly Leu Met Glu Asn Asn Val Leu
65                  70                  75                  80
Val Val

<210> 5
<211> 29
<212> DNA
<213> Artificial Sequence

<220>
<221> source
<222> 1..29
<223> /mol_type="unassigned DNA"
      /note="Arabidopsis thaliana - Primer"
      /organism="Artificial Sequence"

<400> 5
atggatccgc ttctactcga aaacattac                                    29

<210> 6
<211> 32
<212> DNA
<213> Artificial Sequence

<220>
Page 3

SEQLTXT.txt
<221> source
<222> 1..32
<223> /mol_type="unassigned DNA"
      /note="Arabidopsis thaliana - Primer"
      /organism="Artificial Sequence"

<400> 6
tagcggccgc ctattccacc actaaaacat tg                                    32


<210> 7
<211> 29
<212> DNA
<213> Artificial Sequence

<220>
<221> source
<222> 1..29
<223> /mol_type="unassigned DNA"
      /note="Arabidopsis thaliana - Primer"
      /organism="Artificial Sequence"

<400> 7
atgctagctg tgagaaagga atgtctgtg                                        29


<210> 8
<211> 26
<212> DNA
<213> Artificial Sequence

<220>
<221> source
<222> 1..26
<223> /mol_type="unassigned DNA"
      /note="Arabidopsis thaliana - Primer"
      /organism="Artificial Sequence"

<400> 8
atctcgagcg gcgtcgctac gttctc                                           26


<210> 9
<211> 22
<212> DNA
<213> Artificial Sequence

<220>
<221> source
<222> 1..22
<223> /mol_type="unassigned DNA"
      /note="Arabidopsis thaliana - Primer"
      /organism="Artificial Sequence"

<400> 9
atgggatgtg tacccagaat tg                                               22


<210> 10
<211> 24
<212> DNA
<213> Artificial Sequence

<220>
<221> source
<222> 1..24
<223> /mol_type="unassigned DNA"
      /note="Arabidopsis thaliana - Primer"
      /organism="Artificial Sequence"
Page 4

<400> 10
ttccaccact aaaacattgt tctc                                              24


<210> 11
<211> 54
<212> DNA
<213> Artificial Sequence

<220>
<221> source
<222> 1..54
<223> /mol_type="unassigned DNA"
      /note="Oryza sativa - Primer"
      /organism="Artificial Sequence"

<400> 11
ggggacaagt ttgtacaaaa aagcaggctt caccatgtcg gctctcacca cgtc             54


<210> 12
<211> 49
<212> DNA
<213> Artificial Sequence

<220>
<221> source
<222> 1..49
<223> /mol_type="unassigned DNA"
      /note="Oryza sativa - Primer"
      /organism="Artificial Sequence"

<400> 12
ggggaccact ttgtacaaga aagctgggtc aggagcagcc acgttctcc                   49


<210> 13
<211> 57
<212> DNA
<213> Artificial Sequence

<220>
<221> source
<222> 1..57
<223> /mol_type="unassigned DNA"
      /note="Oryza sativa - Primer"
      /organism="Artificial Sequence"

<400> 13
ggggacaagt ttgtacaaaa aagcaggctt caccatggct agaacaatgg gttcaac          57


<210> 14
<211> 49
<212> DNA
<213> Artificial Sequence

<220>
<221> source
<222> 1..49
<223> /mol_type="unassigned DNA"
      /note="Oryza sativa - Primer"
      /organism="Artificial Sequence"

<400> 14
ggggaccact ttgtacaaga aagctgggtc gggagtggcc acgttttcc                   49

<210> 15
<211> 60
<212> DNA
<213> Artificial Sequence

<220>
<221> source
<222> 1..60
<223> /mol_type="unassigned DNA"
      /note="Oryza sativa - Primer"
      /organism="Artificial Sequence"

<400> 15
ggggacaagt ttgtacaaaa aagcaggctt caccatggag tgtctgacca caagttttac     60


<210> 16
<211> 62
<212> DNA
<213> Artificial Sequence

<220>
<221> source
<222> 1..62
<223> /mol_type="unassigned DNA"
      /note="Oryza sativa - Primer"
      /organism="Artificial Sequence"

<400> 16
ggggaccact ttgtacaaga aagctgggtc atttacaaca agtatattgt tctgcattga     60

ac                                                                    62


<210> 17
<211> 26
<212> DNA
<213> Artificial Sequence

<220>
<221> source
<222> 1..26
<223> /mol_type="unassigned DNA"
      /note="Arabidopsis thaliana - Primer"
      /organism="Artificial Sequence"

<400> 17
atggcaactg tgactgcttc ttctaa                                          26


<210> 18
<211> 18
<212> DNA
<213> Artificial Sequence

<220>
<221> source
<222> 1..18
<223> /mol_type="unassigned DNA"
      /note="Arabidopsis thaliana - Primer"
      /organism="Artificial Sequence"

<400> 18
cggcgtcgct acgttctc                                                   18